# EUROPEAN PATENT APPLICATION

(11) **EP 1 757 690 A1**
(43) Date of publication of application: **28.02.2007**
(21) Application number: 05730477.6
(22) Date of filing: 14.04.2005
(51) Int. Cl.: C12N 15/12, C12N 1/21, C12N 5/10, C07K 14/47, C07K 16/18, C12P 21/02, C12Q 1/02, C12Q 1/68, A61K 45/00, A61P 35/00, G01N 33/15, G01N 33/50

(54) **GENE ENCODING GUANINE NUCLEOTIDE EXCHANGE FACTOR AND ITS GENE PRODUCT**

(30) Priority: 15.04.2004 JP 2004120054
(71) Applicant: DAIICHI PHARMACEUTICAL CO., LTD., Chuo-ku, Tokyo 103-8234 (JP); Kazusa DNA Research Institute, Kisarazu-shi, Chiba 292-0818 (JP)
(72) Inventor: OHARA, Osamu, Kazusa DNA Research Inst. Foundation, Kisarazu-shi, Chiba 2920818 (JP); NAGASE, Takahiro, Kazusa DNA Research Inst. Found., Kisarazu-shi, Chiba 2920818 (JP); OHISHI, Michio, Kazusa DNA Research Inst. Found, Kisarazu-shi, Chiba 2920818 (JP); YOKOTA, Hiroshi, Daiichi Pharmaceutical Co., Ltd., Edogawa-ku, Tokyo 1348630 (JP); KAMIDA, Osamu c/o Daiichi Pharmaceutical Co., Ltd., Edogawa-ku, Tokyo 1348630 (JP)
(74) Representative: De Clercq, Ann G. Y.
(86) International application number: PCT/JP2005/007233
(87) International publication number: WO 2005/100567

(57) **Abstract**

A gene encoding a novel protein having a guanine nucleotide exchange factor (GEF) activity for a Rho family protein being one group of small GTP binding proteins, namely, a polynucleotide shown by the nucleotide sequence set forth in SEQ ID NO: 1, SEQ ID NO: 3, or SEQ ID NO: 5, or the complementary strand, the equivalents of the polynucleotide, a protein encoded by the polynucleotide, a vector containing the polynucleotide, a transfomant containing the vector, an antibody against the protein encoded by the polynucleotide, a method of identifying a compound that inhibits the function of the protein encoded by the polynucleotide and/or the expression of the polynucleotide, a method of determining a disease, a pharmaceutical composition, and a reagent kit are provided.

## Description

### TECHNICAL FIELD

The present invention relates to proteins having an activity of a guanine nucleotide exchange factor for Rho family proteins that are one group of small GTP binding protein, and to a polynucleotide encoding the protein. Specifically, the present invention relates to a protein having an activity of a guanine nucleotide exchange factor for a Rho family small GTP binding protein Cdc42, a polynucleotide encoding the protein, a recombinant vector containing the polynucleotide, and a transformant being transfected with the recombinant vector. Further, the present invention relates to a method of producing the protein, and an antibody raised against the protein. Furthermore, the present invention relates to a method of identifying a compound that inhibits the function of the protein and/or the expression of the polynucleotide. Further, the present invention relates to a method of diagnosing a tumor disease such as an esophageal cancer, comprising measuring the amount of expression of the polynucleotide. Furthermore, the present invention relates to an agent for preventing and/or treating a tumor disease such as an esophageal cancer, containing an inhibitor for the function of the protein and/or an inhibitor for the expression of the polynucleotide, and a method of preventing and/or treating a tumor disease such as an esophageal cancer, comprising using an inhibitor for the function of the protein and/or an inhibitor for the expression of the polynucleotide. Further, the present invention relates to a reagent kit containing at least one of the following components: the protein, the polynucleotide, the recombinant vector, the transformant and the antibody.

### BACKGROUND OF INVENTION

A Rho family small GTP binding protein (hereinafter, may be simply referred to as a Rho family protein) belongs to one group of small GTP binding proteins (hereinafter, may be simply referred to as a small G protein). A small G protein works as a signal amplifier between a cell membrane receptor, and an effector participating in an intracellular signal transduction pathway. Further, the small G protein specifically binds to guanosine 5'-triphosphate (GTP) or guanosine 5'-diphosphate (GDP), and shows an enzyme activity of hydrolyzing the bound GTP to GDP. When an extracellular signaling substance binds to a cell membrane receptor, its signal is transduced to a small G protein, which subsequently leads to a reaction exchanging a GDP bound to the small G protein for an intracellular GTP (hereinafter, the reaction may be abbreviated to GDP/GTP exchange reaction). Consequently, an active small G protein that is a GTP binding form is generated. The active small G protein acts on the effector to amplify the signal. Then, the GTP binding form of small G protein hydrolyzes the bound GTP to GDP by its enzyme activity and thereby become inactivated. Thus, the small G protein works as a molecular switch in an intracellular signal transduction pathway by exchanging the guanine nucleotides.

Cdc42, Rac1, RhoA and the like, are known as Rho family proteins. Cdc42 regulates firopodia formation in a fibroblast. Rac1 regulates superoxide production in leukocytes and macrophages, while it regulates cell membrane ruffling and lamellipodia formation in fibroblasts. Further, Cdc42 and Rac1 are capable of activating the c-Jun N-terminal kinase signal transduction pathway. Thus, Rho family proteins are involved in various kinds of cell function by regulating the intracellular signal transduction. For example, cytoskeleton restructuring, cell adhesion, gene expression, and the like, are known as a Rho family protein-mediated cell function. Such functions mediated by Rho family proteins are considered to regulate morphogenesis during ontogeny, migration of leukocyte and the like, axon degeneration, tumor metastasis, and invasion.

Rho guanine nucleotide exchange factor (hereinafter, may be abbreviated as Rho-GEF) is a member of a family of proteins involved in the molecular switching of a Rho family protein. Rho-GEF can function to accelerate the GDP/GTP exchange reaction of a Rho family protein, and can thereby convert the Rho family protein from a GDP-bound inactive form to a GTP-bound active form. Rho-GEF plays an important role through this function, in regulating Rho family protein-mediated intracellular signal transduction. Hereinafter, to accelerate the GDP/GTP exchange reaction and thereby to convert the Rho family protein from a GDP-bound inactive form to a GTP-bound active form may be referred to as a GEF activity or Rho family protein activation.

Rho-GEF has a characteristic domain structure, such as a Dbl homology domain (hereinafter, may be abbreviated as DH domain) and a pleckstrin homology domain (hereinafter, may be abbreviated as PH domain). The DH/PH tandem structure is a typical domain structure for Rho-GEF. Hereinafter, the tandem structure ofDH domain and PH domain may be referred to as DH/PH domain.

The DH/PH domain is an important domain participating in the activation of Rho-GEF, and is considered to be an active domain of Rho-GEF. For example, it has been reported that a protein, which comprises a C-terminal region of the amino acid sequence of proto-Dbl, and contains a DH/PH domain, activated a Rho family protein (Non-Patent Reference 1). proto-Dbl is a prototype of Rho-GEF. Specifically, this report showed that a protein consisting of a C-terminal region within the entire amino acid sequence of proto-Dbl with 925 amino acids, which was generated by deletion of the N-terminal amino acid residues from the 1^{st} to the 497^{th}, activated a Rho family protein, and consequently participated in cellular transformation. From these facts, the activation of proto-Dbl is considered to be an oncogenic activation. Hereinafter, a protein that consists of the C-terminal region of proto-Dbl is referred to as an oncogenic-Dbl. It has been reported that oncogenic-Dbl bind to RhoA, Cdc42 and Rac1, and that they have a GEF activity for Cdc42 and RhoA while they do not exert a GEF activity to Rac1 (Non-Patent Reference 2).

Vav (Non-Patent Reference 3 and 4), ost (Non-Patent Reference 5), lbc (Non-Patent Reference 6), and the like, are known to be genes that encode proto-Dbl family proteins. These genes are known to be involved in cancer. Further, trio (Non-Patent Reference 7), kalirin (Non-Patent Reference 8), and the like, are reported to be genes that encode proteins working as Rho-GEF. A trio knocked-out mouse shows an abnormal skeletal muscle structure and an abnormal brain structure in embryogenesis. Kalirin is involved in axon formation in neurons. Thus, each protein working as Rho-GEF is involved in a cellular function particular to each kind of protein, and activates a different Rho family protein.

Literatures referred in the present description are listed hereunder.
Non-patent Reference 1: Bi, F. et al., Molecular and Cellular Biology, 2001, Vol. 21, p. 1463-1474
Non-patent Reference 2: Hart, M. J. et al., Journal of Biological Chemistry, 1994, Vol. 269, p. 62-65
Non-patent Reference 3: Katzav, S. et al., EMBO Journal, 1989, Vol. 8, p. 2283-2290
Non-patent Reference 4: Costello, P. S. et al., Proceedings of The National Academy of Sciences of The United States of America, 1999, Vol. 96, p. 3035-3040
Non-patent Reference 5: Horii, Y. et al., EMBO Journal, 1994, Vol. 13, p. 4776-4786
Non-patent Reference 6: Toksoz, D. et al., Oncogene, 1994, Vol. 9, p. 621-628
Non-patent Reference 7: O'Brien, S. P. et al., Proceedings of The National Academy of Sciences of The United States of America, 2000, Vol. 97, p. 12074-12078
Non-patent Reference 8: Penzes, P. et al., Journal of Neuroscience, 2001, Vol. 21, p. 8426-8434

### DISCLOSURE OF THE INVENTION

### [PROBLEM TO BE SOLVED BY THE INVENTION]

An object of the present invention is to provide a novel Rho-GEF, and a gene encoding the Rho-GEF. Further, the object of the present invention includes providing a recombinant vector that contains the gene, and a transformant transfected with the recombinant vector. Furthermore, an object of the present invention includes providing a method of producing the Rho-GEF, and an antibody recognizing the Rho-GEF. Further, an object of the present invention includes providing a method of identifying a compound that inhibits the function of the Rho-GEF and/or the expression of the gene. Furthermore, an object of the present invention includes providing a method of preventing and/or treating a disease due to the abnormal function of the Rho-GEF and/or the abnormal expression of the gene, a method of diagnosing the disease, and a reagent kit.

### [MEANS FOR SOLVING THE OBJECT]

The present inventors have concentrated their efforts to meet the aforementioned object. Then, the present inventors have discovered a novel gene having a DH/PH domain-coding region characteristic for Rho GEF, and have successfully obtained the gene. Further, the present inventors revealed experimentally that a protein encoded by a polynucleotide, which is shown by a partial nucleotide sequence of the gene containing a DH/PH domain-coding region, showed a GEF activity for a Rho family protein, Cdc42. Moreover, the present inventors found that the tissue expression of the Rho-GEF gene is approximately 2.5 times or more higher, in an esophageal cancer case compared to that in a normal esophageal tissue. The present invention has been thus achieved.

In various embodiments, the present invention relates to a polynucleotide shown by the nucleotide sequence set forth in SEQ ID NO: 1 or by the complementary nucleotide sequence, or a polynucleotide encoding a protein shown by the amino acid sequence set forth in SEQ ID NO: 2 or a polynucleotide shown by the complementary nucleotide sequence of the polynucleotide.

The present invention also relates to a polynucleotide shown by the nucleotide sequence set forth in SEQ ID NO: 3 or SEQ ID NO: 5 or by the complementary nucleotide sequence, or a polynucleotide encoding a protein shown by the amino acid sequence set forth in SEQ ID NO: 4 or SEQ ID NO: 6 or a polynucleotide shown by the complementary nucleotide sequence of the polynucleotide.

The present invention further relates to a polynucleotide that contains a polynucleotide shown by the nucleotide sequence set forth in SEQ ID NO: 3 or by the complementary nucleotide sequence, or a polynucleotide that contains a polynucleotide encoding a protein shown by the amino acid sequence set forth in SEQ ID NO: 4 or a polynucleotide shown by the complementary nucleotide sequence of the polynucleotide, wherein the polynucleotide encodes a protein having a GEF activity for Cdc42.

The present invention still further relates to a polynucleotide selected from the following group, wherein the polynucleotide encodes a protein having a GEF activity for Cdc42:
(i) a polynucleotide shown by a nucleotide sequence having a homology of at least approximately 70% with the nucleotide sequence of the aforementioned polynucleotide,
(ii) a polynucleotide with a mutation or an induced mutation, such as deletion, substitution, addition of one or several nucleotides in the nucleotide sequence of the aforementioned polynucleotide, and
(iii) a polynucleotide that hybridizes to the aforementioned polynucleotide under stringent conditions.

The present invention also relates to recombinant vectors containing any one of the aforementioned polynucleotides.

The present invention further relates to transformants that have been transfected with any of the aforementioned recombinant vectors.

The present invention still further relates to transformants that have been transfected with any of the aforementioned recombinant vectors and recombinant vectors containing a polynucleotide encoding Cdc42.

The present invention also relates to a protein shown by the amino acid sequence set forth in SEQ ID NO: 2 in the sequence listing.

The present invention further relates to a protein shown by the amino acid sequence set forth in SEQ ID NO: 4 or SEQ ID NO: 6 in the sequence listing.

The present invention still further relates to proteins encoded by any one of the aforementioned polynucleotides.

The present invention also relates to methods of producing any one of the aforementioned proteins, comprising a step of culturing the aforementioned transformants.

The present invention further relates to antibodies that recognize any one of the aforementioned proteins.

The present invention still further relates to a method of identifying a compound that inhibits the function of any one of the aforementioned proteins, and/or the expression of any one of the aforementioned polynucleotides. The method can comprise detecting the presence, absence or change in the function and/or the expression of any of the aforementioned proteins or polynucleotides, under conditions where the interaction of a compound with the protein and/or the polynucleotide are allowed, and determining whether the compound inhibits the function of the protein and/or the expression of the polynucleotide.

The present invention also relates to the aforementioned identification method, wherein the function of the protein is a GEF activity for Cdc42.

The present invention further relates to a method of determining whether a tissue specimen derived from a human esophageal tissue is a tissue derived from a human esophageal cancer or not, comprising measuring an amount of expression of any one of the aforementioned polynucleotides in the tissue specimen.

The present invention still further relates to the aforementioned determination method, wherein the method determines that the tissue specimen is a tissue derived from a human esophageal cancer where the amount of expression of any one of the aforementioned polynucleotides in the tissue specimen is 2.5 times or more higher than that in a control tissue derived from human normal esophagus.

The present invention also relates to agents for preventing and/or treating esophageal cancers, comprising a compound that inhibits the function of any one of the aforementioned proteins, and/or a compound that inhibits the expression of any one of the aforementioned polynucleotides, as an active ingredient.

The present invention further relates to methods of preventing and/or treating esophageal cancers, comprising using a compound that inhibits the function of any one of the aforementioned proteins, and/or a compound that inhibits the expression of any one of the aforementioned polynucleotides.

The present invention still further relates to a reagent kit containing at least one of the aforementioned proteins, the aforementioned polynucleotides, the aforementioned recombinant vectors, the aforementioned transformants, and the aforementioned antibodies.

### ADVANTAGE OF THE INVENTION

The present invention can provide polynucleotides encoding novel proteins that have a GEF activity for Rho family proteins and proteins encoded by the polynucleotides. The present invention can also provide recombinant vectors containing the polynucleotides and transformants transfected with the recombinant vectors. Further, the present invention can provide methods of producing the proteins, and antibodies specific to the proteins. Furthermore, the present invention can provide methods of identifying a compound that inhibits the function of the proteins and/or the expression of the polynucleotides. Further, the present invention can provide methods of diagnosing a tumor disease such as esophageal cancers, comprising measuring an amount of expression of the polynucleotides. Furthermore, the present invention can provide agents for preventing and/or treating a tumor disease such as esophageal cancers, comprising an inhibitor of the function of the proteins and/or an inhibitor of the expression of the polynucleotides, as an active ingredient. Further, the present invention can provide methods of preventing and/or treating a tumor disease such as esophageal cancers, comprising using the inhibitor of the function of the proteins and/or the inhibitor of the expression of the polynucleotides. Furthermore, the present invention can provide a reagent kit comprising at least one of the following components: the protein; the polynucleotide; the recombinant vector; the transformant; and the antibody.

The present protein activated a Rho family protein Cdc42. The present invention allows elucidation and regulation of the signal transduction pathway and cellular function which are mediated by Rho family proteins, for example, Cdc42. Further, the present invention allows diagnosis, prevention and/or treatment of a disease due to an abnormal function of the present proteins and/or an abnormal expression of the present polynucleotides, for example, a tumor disease, more specifically, esophageal cancers.

### BRIEF DESCRIPTION OF THE DRAWINGS

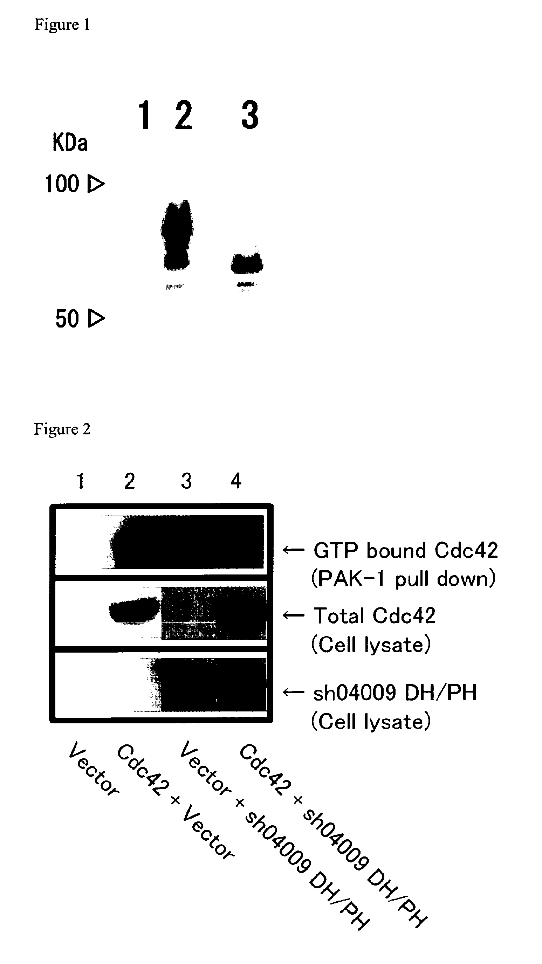

Figure 1 shows a band corresponding to a protein encoded by full-length sh04009 gene (SEQ ID NO: 1), or by DNA (SEQ ID NO: 5) which consists of DNA (sh04009 DH/PH) shown by a partial sequence of the gene containing a DH/PH domain coding region with a kozak sequence and a methionine codon ligated to its 5'-terminal, that was detected (lanes 2 and 3, respectively) by Western blotting of a cell lysate prepared from a cell that was transfected with an expression vector constructed by using the gene, or by using the DNA. Such a band was not detected (lane 1) in a cell lysate prepared in the same manner from a cell that had been added only with Lipofectamine^{™} 2000 instead of transfecting with the vector. (Example 2)

Figure 2 shows, in the upper panel, that the active Cdc42 (GTP bound Cdc42) that binds to PAK-1 was increased in a cell lysate prepared from a cell into which an expression vector constructed by using DNA (SEQ ID NO: 5) which consists of DNA (sh04009 DH/PH) shown by a partial sequence of full-length sh04009 gene containing a DH/PH domain coding region with a kozak sequence and a methionine codon ligated to its 5'-terminal was co-transfected with an expression vector for Cdc42 gene (lane 4), compared to that in a cell lysate prepared from a cell into which only an expression vector for Cdc42 gene was transfected (lane 2), in PAK-1 pull down method. The middle panel shows that a total amount of Cdc42 was almost the same in the cell lysate prepared from the cell into which the expression vector for sh04009DH/PH was co-transfected with the expression vector for Cdc42 gene (lane 4), and in the cell lysate prepared from the cell into which only an expression vector for Cdc42 gene was transfected (lane 2). The lower panel shows that an amount of sh04009DH/PH protein was almost the same in the cell lysate prepared from the cell into which the expression vector for sh04009DH/PH was co-transfected with the expression vector for Cdc42 gene (lane 4), and in a cell lysate prepared from a cell into which only an expression vector for sh04009DH/PH was transfected (lane 3). None of active Cdc42, Cdc42 and sh04009DH/PH protein was detected in a cell lysate prepared from a cell into which only a vector was transfected (lane 1 in the upper, middle and lower panels). (Example 3)

### DETAILED DESCRIPTION OF THE INVENTION

Embodiments of the present invention are explained in further detail below.

In the present invention, the term "polynucleotide" may be used as a generic term which includes the following: an isolated full-length DNA and/or RNA; a synthetic full-length DNA and/or RNA; an isolated DNA oligonucleotide and/or RNA oligonucleotide; or a synthetic DNA oligonucleotide and/or RNA oligonucleotide. Such DNA and/or RNA used herein comprise two or more nucleotides.

In the present invention, the term "protein" may be used as a generic term which includes the following: an isolated or a synthetic full-length protein; an isolated or a synthetic full-length polypeptide; and an isolated or a synthetic full-length oligopeptide. A protein, a polypeptide or an oligopeptide used herein comprises two or more amino acids. Hereinafter, an amino acid may be represented by a single letter or by three letters.

### (polynucleotide)

An aspect of the present invention relates to a novel polynucleotide. The present polynucleotide was identified and isolated from a cDNA library derived from human spleen tissue as a gene having a region encoding a DH/PH domain that is a characteristic domain for Rho-GEF.

The polynucleotide according to the present invention can be a polynucleotide shown by the nucleotide sequence set forth in SEQ ID NO: 1 in the sequence listing, or by the complementary nucleotide sequence thereof. The polynucleotide shown by the nucleotide sequence set forth in SEQ ID NO: 1 is 6018 bp long and contains an open reading flame (ORF) encoding 574 amino acid residues (SEQ ID NO: 2). Further, the polynucleotide shown by the nucleotide sequence set forth in SEQ ID NO: 1 contains a region encoding a DH/PH domain that is an active domain of Rho-GEF. The region in the nucleotide sequence set forth in SEQ ID NO: 1, which consists of the nucleotides from the 496^{th} to the 1032^{nd}, encodes a DH domain consisting of 179 amino acid residues from the 166^{th} valine (Val) to the 344^{th} asparagine (Asn) of the amino acid sequence set forth in SEQ ID NO: 2. The region in the nucleotide sequence set forth in SEQ ID NO: 1, which consists of the nucleotides from the 1132^{nd} to the 1449^{th}, encodes a PH domain consisting of 106 amino acid residues from the 378^{th} leucine (Leu) to the 483^{rd} arginine (Arg) of the amino acid sequence set forth in SEQ ID NO: 2. The region in the nucleotide sequence set forth in SEQ ID NO: 1, which consists of the nucleotides from the 496^{th} to the 1449^{th}, encodes a DH/PH domain consisting of 318 amino acid residues from the 166^{th} valine (Val) to the 483^{rd} arginine (Arg) of the amino acid sequence set forth in SEQ ID NO: 2.

A polynucleotide encoding a protein shown by the amino acid sequence set forth in SEQ ID NO: 2, or a polynucleotide shown by the complementary nucleotide sequence of the polynucleotide is also included in the scope of the present invention.

The embodiment of the polynucleotide according to the present invention can also be a polynucleotide shown by the nucleotide sequence set forth in SEQ ID NO: 3 or SEQ ID NO: 5, or by the complementary nucleotide sequence thereof. The polynucleotide shown by the nucleotide sequence set forth in SEQ ID NO: 3 is a polynucleotide shown by the nucleotide sequence from the 388^{th} nucleotide to the 1722^{nd} nucleotide, of the nucleotide sequence set forth in SEQ ID NO: 1. The polynucleotide shown by the nucleotide sequence set forth in SEQ ID NO: 5 is a polynucleotide which consists of a polynucleotide shown by the nucleotide sequence set forth in SEQ ID NO: 3 and an oligonucleotide (SEQ ID NO: 10) ligated to its 5'-terminal. The oligonucleotide (SEQ ID NO: 10) consists of a kozak consensus sequence (hereinafter, may be referred to as a kozak sequence) and a methionine codon. The polynucleotide shown by the nucleotide sequence set forth in SEQ ID NO: 3, or SEQ ID NO: 5, contains a region encoding a DH/PH domain that is an active domain of Rho-GEF.

The polynucleotide shown by the nucleotide sequence set forth in SEQ ID NO: 5, encodes a protein having a GEF activity for Rho family proteins. Concretely, co-expression of the polynucleotide shown by the nucleotide sequence set forth in SEQ ID NO: 5 with a gene encoding Cdc42 in an animal cell, resulted in increase of active Cdc42 (GTP bound form), compared to an animal cell in which only a gene encoding Cdc42 was expressed (See Example 3). The polynucleotide shown by the nucleotide sequence set forth in SEQ ID NO: 5 is a polynucleotide which consists of a polynucleotide (SEQ ID NO: 3) shown by the nucleotide sequence from the 388^{th} nucleotide to the 1722^{nd} nucleotide of the nucleotide sequence set forth in SEQ ID NO: 1, and an oligonucleotide (SEQ ID NO: 10) ligated to its 5'-terminal. The oligonucleotide (SEQ ID NO: 10) consists of a kozak sequence and a methionine codon. The protein (SEQ ID NO: 6) encoded by the polynucleotide shown by the nucleotide sequence set forth in SEQ ID NO: 5 is a protein (SEQ ID NO: 4) encoded by the polynucleotide shown by the nucleotide sequence set forth in SEQ ID NO: 3, with a methionine added to its N-terminal by peptide bond. Because the oligonucleotide (SEQ ID NO: 10) consisting of a kozak sequence and a methionine codon was added for the purpose of expressing the polynucleotide shown by the nucleotide sequence set forth in SEQ ID NO: 3, it has little effect on the function of the expressed protein. Therefore, the present inventors believe that the polynucleotide shown by the nucleotide sequence set forth in SEQ ID NO: 3, encodes a protein having a GEF activity for Rho family proteins, although it does not have the oligonucleotide (SEQ ID NO: 10) consisting of a kozak sequence and a methionine codon.

The polynucleotides according to the present invention may be polynucleotides that encode proteins having a GEF activity for Rho family proteins or proteins capable of activating Rho family proteins, or polynucleotides shown by the complementary nucleotide sequences of the polynucleotides.

The phrase "GEF activity for Rho family proteins" or "activation of Rho family proteins" means to exchange guanosine 5'-diphosphate (GDP), that is bound to a Rho family protein, for guanosine 5'-triphosphate (GTP), and thereby to convert the Rho family protein from a GDP-bound inactive form to a GTP-bound active form. This exchange reaction comprises a dissociation reaction of GDP from a Rho family protein, and a binding reaction of GTP to the resultant nucleotide free Rho family protein. Specifically, the phrase "activation of Cdc42" or "GEF activity for Cdc42" means to convert Cdc42 from an inactive form to an active form via an exchange reaction of Cdc42-bound GDP for intracellular GTP.

In the present specification, the phrase "GEF activity for Rho family proteins" and the phrase "activation of Rho family proteins" can be used interchangeable.

Proteins encoded by the polynucleotides of the present invention contain a DH/PH domain that is an active domain of Rho-GEF, and activate Rho family proteins. In general, proteins that activate Rho family proteins, namely, proteins having a GEF activity for Rho family proteins, exhibits the function by binding to Rho family proteins. Therefore, the inventors believe that the proteins encoded by the polynucleotides of the present invention bind to Rho family proteins and thereby activate Rho family proteins.

The protein encoded by the polynucleotide shown by the nucleotide sequence set forth in SEQ ID NO: 3, and the protein encoded by the polynucleotide shown by the nucleotide sequence set forth in SEQ ID NO: 5, both have a GEF activity for Rho family proteins as described above. The protein encoded by the polynucleotide shown by the nucleotide sequence set forth in SEQ ID NO: 3 can be, for example, a protein shown by the amino acid sequence set forth in SEQ ID NO: 4. The protein encoded by the polynucleotide shown by the nucleotide sequence set forth in SEQ ID NO: 5 can be, for example, a protein shown by the amino acid sequence set forth in SEQ ID NO: 6. A polynucleotide encoding a protein shown by the amino acid sequence set forth in SEQ ID NO: 4, or SEQ ID NO: 6, or a polynucleotide shown by the complementary nucleotide sequence of the polynucleotide, is also included in the scope of the present invention.

Since the polynucleotide shown by the nucleotide sequence set forth in SEQ ID NO: 3 encodes a protein having a GEF activity for Rho family proteins, the present inventors believe that a polynucleotide containing the polynucleotide shown by the nucleotide sequence set forth in SEQ ID NO: 3, also encodes a protein having a GEF activity for Rho family proteins. Further, the present inventors believe that a polynucleotide containing the polynucleotide encoding the protein shown by the amino acid sequence set forth in SEQ ID NO: 4, also encodes a protein having a GEF activity for Rho family proteins. The polynucleotide containing the polynucleotide shown by the nucleotide sequence set forth in SEQ ID NO: 3 can be, for example, the polynucleotide shown by the nucleotide sequence set forth in SEQ ID NO: 1. The present inventors believe that the polynucleotide shown by the nucleotide sequence set forth in SEQ ID NO: 1, also encodes a protein having a GEF activity for Rho family proteins.

A polynucleotide containing the polynucleotide shown by the nucleotide sequence set forth in SEQ ID NO: 3 or a complementary nucleotide sequence, or a polynucleotide containing the polynucleotide encoding the protein shown by the amino acid sequence set forth in SEQ ID NO: 4 or the polynucleotide shown by the complementary nucleotide sequence of the polynucleotide, are also included in the scope of the present invention. A polynucleotide that also encodes a protein having a GEF activity for Rho family proteins is more preferable for such a polynucleotide. Further, a polynucleotide having a DH/PH domain coding region is still more preferable for such a polynucleotide.

A Rho family protein, which is activated by the proteins encoded by the polynucleotides according to the present invention, may be preferably exemplified by Cdc42 proteins. In the present invention, the phrase "Rho family protein" may preferably direct to Cdc42 proteins. A Rho family protein is not limited to these specific examples and can be any Rho family protein, as long as it is activated by the proteins encoded by the present polynucleotides. The activation of a Rho family protein by the proteins encoded by the present polynucleotides can be measured by using, for example, an effector pull-down assay (see Example 3).

The amino acid sequence of Cdc42 and the nucleotide sequence of Cdc42 gene are set forth in SEQ ID NO: 12 and SEQ ID NO: 11, respectively. Cdc42 and its gene are not limited to the specific examples shown by the aforementioned sequences. They can be a protein or a gene with a mutation of one or several sites in the aforementioned sequence, as long as it has a function of Cdc42 generally known. Further, a mutant which is prepared by introducing a mutation into one or several sites of the aforementioned sequence can be used to enhance or diminish the function of the protein and the gene. Cdc42 can be produced, for example, by culturing a transformant that was prepared by transfecting with a recombinant vector containing the gene by using well-known gene manipulation techniques.

Polynucleotides according to the present invention can be prepared based on the sequence information concerning the specific example provided by the present invention, such as the polynucleotide shown by the nucleotide sequence set forth in SEQ ID NO: 1 in the sequence listing. The production of the polynucleotide can be carried out easily by using gene manipulation techniques that are well known per se (Sambrook et al., Eds., "Molecular Cloning, A Laboratory Manual, 2nd Edition", 1989, Cold Spring Harbor Laboratory: Muramatsu Masami., Ed., "Labomanual Genetic Engineering", 1988, Maruzen Co., Ltd.).

Specifically, the polynucleotide of the present invention can be acquired by preparing a cDNA library in accordance with an ordinary method, from a suitable source in which expression of the polynucleotide was found, and then selecting a desired clone from the cDNA library. As a cDNA source, various kinds of cells and tissues in which expression of the present polynucleotide was found, or cultured cells derived from these cells and tissues, for example, cells derived from human spleen tissue, or the like, can be used. Isolation of total RNA from these sources, isolation and purification of mRNA, acquisition of cDNA, the cloning thereof, and the like, can each be performed in accordance with an ordinary method. It is also possible to use a cDNA library that was constructed from commercially available polyA⁺ RNA derived from human spleen tissue. A method for selecting a desired clone from a cDNA library is not particularly limited, and any methods generally used can be employed. For example, selection of a desired clone can be performed by using a probe or primer capable of selectively hybridizing to the present polynucleotide. Specifically, a plaque hybridization method, colony hybridization method, or the like, which uses a probe capable of selectively hybridizing to the present polynucleotide, or a combination of these methods, can be employed. As a probe, a polynucleotide chemically synthesized based on the sequence information of the present polynucleotide, and the like, can generally be used. The present polynucleotide prepared, or a polynucleotide shown by the partial nucleotide sequence of the present polynucleotide, is suitable for use as a probe, as well. Furthermore, a sense primer and an anti-sense primer, which were designed based on the sequence information of the present polynucleotide, can also be used as such a probe.

Selection of a desired clone from a cDNA library can be performed, for example, by detecting the expression of the protein in each clone, utilizing a known protein expression system, and further determining a biological function of the protein as an indicator. As a function of the protein encoded by the present polynucleotide, for example, the function of activating Rho family proteins such as Cdc42, and the like, namely, a GEF activity for Rho family proteins can be mentioned. Any known expression system can be used as a protein expression system. For example, a cell free protein expression system can be conveniently used as such a system (Madin, K. et al., Proceedings of The National Academy of Sciences of The United States of America, 2000, Vol. 97, p.559-564).

The polynucleotide according to the present invention can be also prepared preferably by using a DNA/RNA amplification method. For example, a polymerase chain reaction can be used (hereinafter, may be abbreviated as PCR: Ulmer, K.M. Science, 1983, Vol. 219, p.666-671; Ehrlich, H. A., Ed., PCR Technology. Principles and Applications for DNA Amplification, 1989, Stockton Press; and Saiki, R.K. et al., Science, 1985, Vol. 230, p.1350-1354). When the full-length cDNA is difficult to obtain from a cDNA library, a RACE method (Jikken Igaku (Experimental Medicine), 1994, Vol. 12, No. 6, p. 615-618), particularly the 5'-RACE method (Frohman, M. A. et al., Proceedings of The National Academy of Sciences of The United States of America, 1988, Vol. 85, No. 23, p.8998-9002), or the like, can be suitably employed. Primers to be used for PCR can be suitably designed based on the nucleotide sequence information of the polynucleotide, and can be obtained by synthesis in accordance with any conventional method. Isolation and purification of amplified DNA/RNA fragments can be carried out according to any conventional method, such as gel electrophoresis, or the like.

A determination of the nucleotide sequence of polynucleotide can be carried out by any conventional method, such as the dideoxy method (Sanger, F. et al., Proceedings of The National Academy of Sciences of The United States of America, 1977, Vol. 74, p.5463-5467), and the Maxam-Gilbert method (Maxam, A.M. et al., Methods in Enzymology, 1980, Vol. 65, p.499-560), or by simply using a commercially available sequencing kit, or the like.

The polynucleotides according to the present invention are not limited to the aforementioned polynucleotides, and can include polynucleotides having a sequence homology with the aforementioned polynucleotides and encoding proteins having a GEF activity for Rho family proteins, or polynucleotides shown by the complementary nucleotide sequences of the polynucleotides. A suitable sequence homology with the entire base sequence is normally 50% or more, preferably at least 70%. It is more preferably 70% or more, even more preferably 80% or more, and still more preferably 90% or more. Further, polynucleotides having a DH/PH domain coding region are still more preferable for such polynucleotides. A suitable sequence homology with the DH/PH domain coding region is preferably at least 70%. It is more preferably 70% or more, even more preferably 80% or more, and still more preferably 90% or more. In addition, it is still more preferable that the DH/PH domain has its specific function such as a GEF activity for Rho family proteins.

The polynucleotides of the present invention include polynucleotides shown by a nucleotide sequence with a mutation, such as deletion, substitution, addition or insertion, of one or more nucleotides in the nucleotide sequences of the aforementioned DNA, or polynucleotides shown by complementary nucleotide sequences. The number of mutated nucleotides is, for example, from 1 to 100, preferably from 1 to 30, more preferably from 1 to 20, even more preferably from 1 to 10, and still more preferably from 1 to several in number. The extent of mutation, the position of a mutation, and the like, are not particularly limited, as long as the polynucleotides with mutations encode proteins having a GEF activity for Rho family proteins, and/or preferably proteins having DH/PH domain. The polynucleotides with mutations may be natural polynucleotides, or may be mutated polynucleotides. Further, they also may be polynucleotides prepared by introducing a mutation into a natural gene. Such a polynucleotide may be, for example, a polynucleotide with a substitution of the 1566^{th} thymine (T) to cytosine (C) in the nucleotide sequence set forth in SEQ ID NO: 1. Techniques for introducing a mutation are known in the art. For example, site-directed mutagenesis, genetic homologous recombination, primer extension, PCR, and the like, can be used independently or in suitable combinations. Specifically, for example, a method described in publications (Sambrook et al., Eds., "Molecular Cloning, A Laboratory Manual, 2nd Edition", 1989, Cold Spring Harbor Laboratory; and Muramatsu Masami., Ed., "Labomanual Genetic Engineering", 1988, Maruzen Co., Ltd.) or a modified method thereof, can be used for conducting the introduction of a mutation. In addition, Ulmer's techniques (Ulmer, K.M. Science, 1983, Vol. 219, p.666-671) can also be utilized.

The polynucleotides of the present invention can also be polynucleotides that hybridize to the aforementioned polynucleotides under stringent conditions. A hybridization condition can be found, for example, in a method described in publications (Sambrook et al., Eds., "Molecular Cloning, A Laboratory Manual, 2nd Edition", 1989, Cold Spring Harbor Laboratory), or the like. More specifically, the phrase "under stringent conditions" refers to, for example, a condition of heating at 42 °C in a solution containing 6× SSC, 0.5% SDS and 50% formamide, and then washing at 68 °C in a solution containing 0.1 × SSC and 0.5% SDS. Such polynucleotides are not required to have complementary sequences with the present polynucleotides, as long as they hybridize to the present polynucleotides. It is desirable that the encoding proteins are preferably proteins having a GEF activity for Rho family proteins, and/or more preferably proteins having a DH/PH domain.,

The polynucleotides of the present invention also include oligonucleotides shown by a partial nucleotide sequence of a given region of the aforementioned polynucleotides. The minimum unit of such an oligonucleotide consists of consecutive nucleotides within the region, preferably of 5 or more, more preferable of 10 or more, and even more preferably of 20 or more consecutive nucleotides. These oligonucleotides can be prepared by designing a desired sequence based on the nucleotide sequence information of the present polynucleotides, and then synthesizing them using a well-known chemical synthesis method. An automated DNA/RNA synthesizer can be conveniently used for preparing the oligonucleotides. These oligonucleotides can be used, for example, as primers for amplifying the present genes or the present gene fragments, and as probes for detecting the present genes or their transcription products thereof.

Oligonucleotides shown by a partial nucleotide sequence with a given region of the polynucleotides according to present invention can be preferably exemplified by an oligonucleotide shown by the nucleotide sequence set forth in SEQ ID NO: 7, SEQ ID NO: 8, or SEQ ID NO: 9, in the sequence listing.

The polynucleotides of the present invention are preferably human derived polynucleotides. However, the present invention includes polynucleotides derived from mammals, for example, a polynucleotide derive from mouse, horse, sheep, cow, dog, monkey, cat, bear, rat, rabbit or the like, as long as the polynucleotide is a polynucleotide that has a sequence homology with the present polynucleotides, and that encodes a protein having a GEF activity for Rho family proteins. A suitable sequence homology with the entire base sequence is normally 50% or more, preferably at least 70%. It is more preferably 70% or more, even more preferably 80% or more, and still more preferably 90% or more. Further, polynucleotides having a DH/PH domain coding region are more preferable for such polynucleotides. A suitable sequence homology with the DH/PH domain coding region is preferably at least 70%. It is more preferably 70% or more, even more preferably 80% or more, and still more preferably 90% or more.

The polynucleotides of the present invention also may have a desired gene at the 5'-terminal side or the 3'-terminal side thereof, as long as the expression of the polynucleotides or the function of proteins encoded by the polynucleotides, for example, a GEF activity for Rho family proteins, is not inhibited. A gene capable of being added to the present polynucleotide can be specifically exemplified by the genes of enzymes, such as glutathione S-transferase (GST), β-galactosidase, horseradish peroxidase (HRP) or alkaline phosphatase (ALP), or of tag peptides such as His-tag, Myc-tag, HA-tag, FLAG-tag or Xpress-tag. One of these genes, or multiple kinds of these genes in combination, can be added to the present polynucleotides. Addition of these genes can be performed by using conventional gene manipulation techniques, and is useful to facilitate detection of a gene or mRNA.

### (vector)

Another aspect of the present invention relates to recombinant vectors containing a polynucleotide of the present invention. The recombinant vectors can be prepared by inserting the present polynucleotides into a suitable vector DNA.

The vector DNA is not particularly limited, as long as it can be replicated within a host, and can be suitably selected in accordance with the kind of host and purpose of use. The vector DNA may be vector DNA obtained by extracting natural DNA, or may be vector DNA lacking a part of DNA other than a segment necessary for replication. Typical vector DNAs include, for example, a vector DNA derived from a plasmid, a bacteriophage or a virus. A plasmid DNA can be exemplified by a plasmid derived from *Escherichia coli,* a plasmid derived from *Bacillus subtilis,* or a plasmid derived from yeast. A bacteriophage DNA can be exemplified by a λ phage. Vector DNA derived from a virus can be exemplified by a vector derived from an animal virus, such as a retrovirus, vaccinia virus, adenovirus, papovavirus, SV 40, fowlpox virus, and pseudorabies virus, or a vector derived from an insect virus such as baculovirus. Further, vector DNA derived from a transposon, an insertion element, a yeast chromosome element, or the like, may be exemplified. Alternatively, a vector DNA prepared by combining two or more of these, for example, a vector DNA (cosmid, phagemid or the like) prepared by combining genetic elements of a plasmid and a bacteriophage, may be exemplified.

Any vector DNA can be used in accordance with the desired purpose, for example, an expression vector, cloning vector or the like. The recombinant expression vector containing the polynucleotide of the present invention is useful for the production of proteins encoded by the present polynucleotides.

It is necessary for the polynucleotide of the present invention to be incorporated into vector DNA in such a way as to allow the function of the polynucleotide to appear. The vector DNA contains at least one of the present polynucleotides and a promoter, as construction elements. In addition to these elements, as desired, a genetic sequence that encodes information relating to replication and control, may be incorporated in combination into the vector DNA, by using a well-known method. Such a genetic sequence can be exemplified by a ribosome binding sequence, terminator, signal sequence, cis element such as an enhancer, splicing signal, and a selective marker such as dihydrofolate reductase gene, ampicillin-resistant gene and neomycin-resistant gene. The vector DNA may contain one or more kinds of genetic sequences selected from the aforementioned members.

As a method of incorporating the polynucleotide, according to the present invention, into a vector DNA, any known method can be employed. For example, a method may be used which comprises cleaving a gene containing the present polynucleotide at specific sites, by treating it with suitable restriction enzymes, and then mixing it with a similarly treated vector DNA, for ligation using a ligase. Alternatively, a desired recombinant vector may be prepared by using a method that comprises ligating one of the present polynucleotides with a suitable linker, and then inserting it into the multi-cloning site of a vector, suitable for the desired purpose.

### (transformant)

Further aspect of the present invention relates to transformants obtained by transforming a host with the recombinant vectors according to the present invention. A transformant, prepared by introducing a recombinant expression vector that contains a polynucleotide according to the present invention, is useful for producing a protein encoded by the present polynucleotides. The present transformants may further incorporate one or more kinds of vector DNAs, each containing a desired gene other than the present polynucleotides. A vector DNA that contains a desired gene other than a present polynucleotide, can be exemplified by vector DNA that contains a gene encoding a Rho family protein, such as, Cdc42, or the like. A transformant prepared by transforming with both of the expression vectors, one of which contains a present polynucleotide and the other contains a gene encoding a Rho family protein, may be used for a method of identifying a compound that inhibits or enhances a GEF activity of a protein encoded by a present polynucleotide for Rho family proteins. That is to say, the transformant may be used for a method of identifying a compound that inhibits or enhances the activation of Rho family proteins. Such a transformant can be preferably exemplified by a transformant prepared by transforming with both recombinant vectors, one of which contains the present polynucleotide and the other contains a gene encoding Cdc42.

Any suitable prokaryotes and eukaryotes can be employed as a host. Examples of suitable prokaryotes include bacteria belonging to the *Escherichia* genus, such as, *Escherichia coli,* bacteria belonging to the *Bacillus* genus, such as, *Bacillus subtilis,* bacteria belonging to the *Pseudomonas* genus, such as, *Pseudomonas putida,* and bacteria belonging to the *Rhizobium* genus, such as, *Rhizobium meliloti.* Examples of suitable eukaryotes include yeasts, and animal cells such as insect cells and mammalian cells. Yeasts can be exemplified by *Saccharomyces cerevisiae* and *Schizosaccharomyces pombe.* Insect cells can be exemplified by Sf9 cells and Sf2 cells. Mammalian cells can be exemplified by monkey kidney-derived cells, such as COS cells, Vero cells, Chinese hamster ovary cells (CHO cell), mouse L cells, rat GH3 cells, human FL cells, and human 293EBNA cells. It is preferable to use mammalian cells, and more preferable to use 293EBNA cells.

Transformation of a host cell with a recombinant vector can be carried out by utilizing known methods. For example, a standard method described in publications (Sambrook et al., Eds., "Molecular Cloning, A Laboratory Manual, 2nd Edition", 1989, Cold Spring Harbor Laboratory) may be utilized. When gene stability is a consideration, it is preferable to use a method that integrates the gene onto a chromosome. Meanwhile, it is convenient to use an autonomous replication system that utilizes an extranuclear gene. Specifically, calcium phosphate transfection, DEAE-dextran mediated transfection, microinjection, cationic lipid-mediated transfection, electroporation, transduction, scrape loading, ballistic introduction, infection, and the like, may be mentioned.

When employing a prokaryote as a host, it is preferable to use a recombinant vector which is capable of autonomous replication within the bacterium, and is also composed of a promoter, a ribosomal binding sequence, the polynucleotide of the present invention, and a transcription termination sequence. It may also contain a gene that regulates the promoter. When employing bacteria as a host, any promoter may be used, as long as it can lead to expression in bacteria, such as *Escherichia coli.* For example, a promoter derived from *Escherichia coli* or a phage can be used, such as a trp promoter, lac promoter, PL promoter or PR promoter. An artificially designed and modified promoter such as a tac promoter may also be used. A method of introducing a recombinant vector into bacteria is not particularly limited, and any methods that introduce DNA into bacteria can be employed. Preferable examples of such a method include using calcium ions, electroporation, or the like.

When employing a mammalian cell as a host, it is preferable to use the recombinant vector which is capable of autonomous replication within the cell, and is also composed of a promoter, RNA splice site, a polynucleotide of the present invention, polyadenylated site and a transcription termination sequence. As desired, it may also contain an origin of replication. A SRαpromoter, SV 40 promoter, LTR promoter, CMV promoter, and the like, can be used as a promoter. An early gene promoter of cytomegalovirus, and the like, may be used, as well. As a method of introducing the recombinant vector into a mammalian cell, preferably, for example, electroporation, the calcium phosphate technique, lipofection, or the like, may be used. A most preferable method to be used may be lipofection.

When using yeast as a host, the promoter is not particularly limited, as long as it can lead to expression in yeast. Examples of such a promoter include the gal1 promoter, gal10 promoter, heat shock protein promoter, MFα1 promoter, PHO5 promoter, PGK promoter, GAP promoter, ADH promoter, and AOX1 promoter. A method of introducing a recombinant vector into yeast is not particularly limited, as long as it is a method that introduces the DNA into the yeast. Preferable examples of such a method include electroporation, a spheroplast method, a lithium acetate method or the like.

When using an insect cell as a host, it is preferable to use a calcium phosphate technique, lipofection, or electroporation for a method of introducing a recombinant vector.

### (protein)

A further aspect of the present invention relates to proteins encoded by a polynucleotide according to the present invention.

The protein according to the present invention can be, for example, a protein encoded by the polynucleotide shown by the nucleotide sequence set forth in SEQ ID NO: 1. More specifically, the protein can be a protein shown by the amino acid sequence set forth in SEQ ID NO: 2. The protein shown by the amino acid sequence set forth in SEQ ID NO: 2 has a DH domain composed of the amino acid residues from the 166^{th} valine (Val) to the 344^{th} asparagine (Asn). The protein has a PH domain composed of the amino acid residues from the 378^{th} leucine (Leu) to the 483^{rd} arginine (Arg). Thus, the protein has a DH/PH domain composed of the amino acid residues from the 166^{th} valine (Val) to the 483^{rd} arginine (Arg).

An embodiment of a protein according to the present invention can also be a protein encoded by the polynucleotide shown by the nucleotide sequence set forth in SEQ ID NO: 3 or SEQ ID NO: 5. More specifically, the protein encoded by the polynucleotide shown by the nucleotide sequence set forth in SEQ ID NO: 3 can be a protein shown by the amino acid sequence set forth in SEQ ID NO: 4. In addition, the protein encoded by the polynucleotide shown by the nucleotide sequence set forth in SEQ ID NO: 5 can be a protein shown by the amino acid sequence set forth in SEQ ID NO: 6. The amino acid sequence set forth in SEQ ID NO: 4 corresponds to the amino acid sequence set forth in SEQ ID NO: 2 of from the 130^{th} glutamic acid (Glu) to the 574^{th} lysine (Lys). In addition, the amino acid sequence set forth in SEQ ID NO: 6 is an amino acid sequence comprising the amino acid sequence set forth in SEQ ID NO: 4, with a methionine added to its N-terminal by peptide bond. Namely, the protein shown by each of these amino acid sequences contains a DH/PH domain.

A protein encoded by the polynucleotide shown by the nucleotide sequence set forth in SEQ ID NO: 5 has a GEF activity for a Rho family protein. Concretely, co-expression of the polynucleotide shown by the nucleotide sequence set forth in SEQ ID NO: 5, with a gene encoding Cdc42 in an animal cell, resulted in increase of active Cdc42 (GTP bound form), compared to an animal cell in which only a gene encoding Cdc42 was expressed (See Example 3). A protein encoded by the polynucleotide shown by the nucleotide sequence set forth in SEQ ID NO: 5 is a protein which consists of a protein encoded by the polynucleotide shown by the nucleotide sequence set forth in SEQ ID NO: 3 and a methionine added to its N-terminal by peptide bond. The protein encoded by the polynucleotide shown by the nucleotide sequence set forth in SEQ ID NO: 5, is a protein obtained by using a polynucleotide shown by the nucleotide sequence set forth in SEQ ID NO: 3, to which an oligonucleotide (SEQ ID NO: 10) consisting of a kozak sequence and a methionine codon are linked at its 5'-terminal, for the purpose of expressing the polynucleotide shown by the nucleotide sequence SEQ ID NO: 3. The added methionine does not affect the function of the expressed protein. Therefore, the present inventors believe that the protein encoded by the polynucleotide shown by the nucleotide sequence set forth in SEQ ID NO: 3, has a GEF activity for a Rho family protein, though it does not have a methionine at its N-terminal.

A protein according to the present invention may be a protein having a GEF activity for Rho family proteins or a protein capable of activating Rho family proteins.

A protein according to the present invention contains a DH/PH domain that is an active domain of Rho-GEF, and activated a Rho family protein. In general, a protein that activates a Rho family protein, namely, a protein having a GEF activity for a Rho family protein, exhibits the function by binding to a Rho family protein. Therefore, the inventors believe that the present protein binds to a Rho family protein and thereby activates a Rho family protein.

Thus, the protein encoded by the polynucleotide shown by the nucleotide sequence set forth in SEQ ID NO: 3, is believed to have a GEF activity for a Rho family protein in a similar manner as the protein encoded by the polynucleotide shown by the nucleotide sequence set forth in SEQ ID NO: 5. In addition, a DH/PH domain that is contained in these proteins are known to be an important domain concerning to a GEF activity for a Rho family protein. Therefore, a protein that contains the protein encoded by the polynucleotide shown by the nucleotide sequence set forth in SEQ ID NO: 3, is believed to also have a GEF activity for a Rho family protein. Such a protein can be a protein encoded by a polynucleotide that contains the polynucleotide shown by the nucleotide sequence set forth in SEQ ID NO: 3. Further, a protein encoded by a polynucleotide that contains the polynucleotide encoding the protein shown by the amino acid sequence set forth in SEQ ID NO: 4, can be an example of such a protein. Specifically, a protein encoded by a polynucleotide that contains the polynucleotide shown by the nucleotide sequence set forth in SEQ ID NO: 3, may be exemplified by a protein encoded by the polynucleotide shown by the nucleotide sequence set forth in SEQ ID NO: 1. A protein encoded by the polynucleotide shown by the nucleotide sequence set forth in SEQ ID NO: 1, can be exemplified by a protein shown by the amino acid sequence set forth in SEQ ID NO: 2. All of these proteins exemplified herein are believed to have a GEF activity for a Rho family protein.

The present invention includes a protein that is encoded by a polynucleotide that contains the polynucleotide shown by the nucleotide sequence set forth in SEQ ID NO: 3, or by its complementary nucleotide sequence, or that is encoded by a polynucleotide that contains the polynucleotide encoding the protein shown by the amino acid sequence set forth in SEQ ID NO: 4, or by its complementary nucleotide sequence, each of which is a polynucleotide encoding a protein that have a GEF activity for a Rho family protein.

The protein according to the present invention is not limited to a protein as exemplified above, and any protein is included in the scope of the present invention, as long as it is encoded by a polynucleotide according to the present invention. Preferably, a protein that is encoded by a present polynucleotide and that has a GEF activity for Rho family proteins may be mentioned. Such a protein can be exemplified by a protein that is encoded by a polynucleotide shown by a nucleotide sequence having a homology of at least 70% with the nucleotide sequence of any one of the polynucleotides selected from the group consisting of the following: a polynucleotide shown by the nucleotide sequence set forth in SEQ ID NO: 1, or its complementary nucleotide sequence; a polynucleotide encoding the protein shown by the amino acid sequence set forth in SEQ ID NO: 2, or a polynucleotide shown by the complementary nucleotide sequence of the polynucleotide; a polynucleotide shown by the nucleotide sequence set forth in SEQ ID NO: 3, or SEQ ID NO: 5, or by a complementary nucleotide sequence; and a polynucleotide encoding a protein shown by the amino acid sequence set forth in SEQ ID NO: 4, or SEQ ID NO: 6, or a polynucleotide shown by the complementary nucleotide sequence of the polynucleotide, where the protein has a GEF activity for a Rho family protein. Further, the present protein also includes a protein that is encoded by a polynucleotide shown by a nucleotide sequence with a mutation or a induced mutation, such as deletion, substitution, addition or the like, of one or more nucleotides in the nucleotide sequence of any one of polynucleotides selected from the aforementioned polynucleotide group, where the protein is encoded by a polynucleotide encoding a protein that has a GEF activity for a Rho family protein. Furthermore, the present protein may also be exemplified by a protein encoded by a polynucleotide that hybridizes to any one of the polynucleotides selected from the aforementioned polynucleotide group, under stringent conditions, and encodes a protein that has a GEF activity for a Rho family protein.

A protein according to the present invention can be more specifically, for example, a protein that has a sequence homology with the protein shown by the amino acid sequence set forth in SEQ ID NO: 2, SEQ ID NO: 4, or SEQ ID NO: 6, and that has a GEF activity for a Rho family protein. A suitable sequence homology with the entire amino acid sequence is normally 50% or more, preferably at least 70%. It is more preferably 70% or more, even more preferably 80% or more, and still more preferably 90% or more. Further, a polypeptide having a DH/PH domain is still more preferable. A suitable sequence homology with the DH/PH domain is preferably at least 70%. It is more preferably 70% or more, even more preferably 80% or more, and still more preferably 90% or more. In addition, it is still more preferable that the DH/PH domain has a specific function such as a GEF activity for a Rho family protein. Further, a protein of the present invention includes a protein that is shown by an amino acid sequence with a mutation, such as deletion, substitution, addition, or the like, of one or more amino acids in the amino acid sequence set forth in SEQ ID NO: 2, SEQ ID NO: 4, or SEQ ID NO: 6, and that has a GEF activity for a Rho family protein. The mutation of amino acids is, for example 1 to 100, preferably 1 to 30, more preferably 1 to 20, further preferably 1 to 10, and still more preferably 1 to several in number. The extent of mutation, the position of a mutation, and the like, of the amino acid are not particularly limited, as long as a protein with a mutation has a GEF activity for a Rho family protein, and preferably is a protein having a DH/PH domain. Such a protein with a mutation may be a protein generated in nature, for example, due to mutation or post translational modification. Further, it also may be a protein prepared by introducing a mutation into a natural gene. Techniques for introducing a mutation are known in the art. For example, known gene manipulation techniques can be used for preparation. When introducing a mutation, in view of avoiding a change in the fundamental properties (such as physical properties, function, physiological activity, and immunological activity) of the protein, mutual substitution among homologous amino acids (polar amino acids, non-polar amino acids, hydrophobic amino acids, hydrophilic amino acids, positively-charged amino acids, negatively-charged amino acids and aromatic amino acids or the like) may be readily conceived.

A protein according to the present invention further includes a protein shown by a partial sequence of the aforementioned protein. For example, a protein that is shown by a partial sequence of the protein shown by the amino acid sequence set forth in SEQ ID NO: 2, SEQ ID NO: 4, or SEQ ID NO: 6, is also included in the scope of the present invention. The minimum unit of such a protein consists of consecutive amino acids, preferably 5 or more, more preferably 8 or more, even more preferably 12 or more, or 15 or more.

A protein of the present invention is preferably a human derived protein. However, the present invention includes proteins derived from mammals, for example, proteins derived from a mouse, horse, sheep, cow, dog, monkey, cat, bear, rat, rabbit, or the like, as long as the protein is a protein that has a sequence homology with the present proteins, and that has a GEF activity for a Rho family protein. Further, a protein having a DH/PH domain is more preferable for such a protein. A suitable sequence homology with the entire amino acid sequence is normally 50% or more, preferably at least 70%. It is more preferably 70% or more, even more preferably 80% or more, and still more preferably 90% or more. A suitable sequence homology with the DH/PH domain is preferably at least 70%. It is more preferably 70% or more, even more preferably 80% or more, and still more preferably 90% or more.

A protein of the present invention may be a protein prepared from a cell in which a gene encoding the present protein is expressed by means of a gene manipulation technique, or from any suitable biological sample. A protein also may be a product of a cell-free synthesis system, or a chemical synthesis product. These can be subsequently further purified for use. In addition, the present protein may be a protein being expressed in a cell that contains a gene encoding the present protein. The cell may be a transformant prepared by transfecting a vector that contains a gene encoding the present protein.

A protein of the present invention can be modified to the extent that no significant functional change is involved, such as amidation of its constituent amino groups, carboxyl groups, or the like. A present protein can also be labeled with the other protein, or the like, that is added to the N-terminal or C-temlinal, directly or indirectly, via a linker peptide, or the like, or by means of gene manipulation techniques, or the like. Labeling is preferably conducted in a way not to inhibit the fundamental properties of the present protein. Even more preferably, labeling is conducted in a way not to inhibit GEF activity of the present protein for a Rho family protein. A substance used for labeling (a labeling substance) can be exemplified by enzymes such as GST, β-Gal, HRP, ALP, or the like, tag peptides such as His-tag, Myc-tag, HA-tag, FLAG-tag, Xpress-tag or the like, fluorescent substances, such as fluorescein isothiocyanate, phycoerythrin or the like, a maltose binding protein, an immunogloblin Fc-fragment, biotin, or the like. However, it is not limited to these specific examples. Labeling can also be carried out using a radioactive isotope. One or more kinds of labeling substances in combination can be added to the present protein. These labeling substances allow the detection and/or purification of the present protein to become easier, by measuring the substance itself, or the function thereof. In addition, these substances allow, for example, the detection of the binding of the present protein to the other protein, and the measurement of the function of the present protein.

### (Method of producing a protein)

A further aspect of the present invention relates to a method of producing a protein according to the present invention. The present protein can be prepared, for example, by standard gene manipulation techniques (refer to Sambrook et al., Eds., "Molecular Cloning, A Laboratory Manual, 2nd Edition", 1989, Cold Spring Harbor Laboratory; Muramatsu Masami., Ed., "Labomanual Genetic Engineering", 1988, Maruzen Co., Ltd.; Ulmer, K.M. Science, 1983, Vol. 219, p.666-671; Ehrlich, H. A., Ed., PCR Technology. Principles and Applications for DNA Amplification, 1989, Stockton Press and the like,) based on the nucleotide sequence information of a gene encoding the present protein. For example, a cDNA library may first be prepared from various kinds of cells or tissues in which the expression of the present polynucleotide was found, or cultured cells derived from these cells and tissues, in accordance with conventional methods. Then, the polynucleotide encoding the present protein may be amplified from the cDNA library, by using a primer that selectively hybridizes to the gene encoding the protein. The amplified polynucleotide may be subjected to the expression induction by using known gene manipulation techniques to produce the present protein.

Specifically, for example, the present proteins can be produced by culturing the transformants according to the present invention, and then collecting the present proteins from the culture product obtained. The transformant can be cultured according to known culture conditions and culture methods that are suitable for a host used for the preparation of the transformant. Cultivation can be carried out by employing an indicator, such as the present proteins themselves that are expressed by the transformant, or a function thereof. For example, a function can be a GEF activity for a Rho family protein. Alternatively, cultivation may be carried out by employing such an indicator as the present proteins themselves produced in a host or outside a host, or the amount of the protein. Otherwise, subculturing or batch culturing may be carried out by employing such an indicator as the amount of transformant in the culture medium.

When the proteins according to the present invention are expressed in a transformant, or on its cell membrane, the proteins may be extracted from the disrupted transformant. Further, when the present proteins are secreted outside the transformant, the cultured medium can be used as is, or the cultured medium can be used after removing the transformant by centrifugation or the like. As desired, the present protein can be isolated and/or purified from a cultured medium of the transformant or from the transformant.

Purification and/or isolation of the present protein can be carried out by various isolation methods that utilize the physical properties or chemical properties of the present protein. Specific examples of the isolation methods may include ammonium sulfate precipitation, ultrafiltration, gel chromatography, ion-exchange chromatography, affinity chromatography, high performance liquid chromatography, and dialysis. These methods may be used in suitable combinations. It is preferable to employ an affinity chromatography that utilizes a column bound with a specific antibody specific to the present protein. The specific antibody to the present protein can be prepared based on the amino acid information of the protein by a well known method of producing an antibody. The purification and/or isolation may be carried out by employing an indicator to obtain the present protein, such as a function of the present protein, for example, a GEF activity for a Rho family protein.

The proteins of the present invention can also be produced according to conventional chemical synthesis methods. As a chemical synthesis method of a protein, for example, methods described in publications ("peptide synthesis", Maruzen Co., Ltd., 1975; and "peptide synthesis", Interscience, New York, 1996) may be exemplified. However, it is not limited to these methods, and any known methods can be used. Specifically, .solid phase synthesis, solution phase synthesis, and the like, are known as chemical synthesis methods for proteins, and any of these methods can be used. These kinds of protein synthesis methods more specifically include a so-called stepwise elongation method that sequentially binds each amino acid, one at a time, to elongate a chain based on amino acid sequence information, and a fragment condensation method that previously synthesizes fragments consisting of several amino acids, and subsequently subjects the respective fragments to a coupling reaction. The present proteins can be synthesized by either of these methods. A condensation method used for the aforementioned protein synthesis methods can also be carried out according to conventional methods. Examples of condensation methods include an azide method, mixed anhydride method, DCC method, active ester method, oxidation-reduction method, DPPA (diphenylphosphoryl azide) method, DCC + additive (1-hydroxybenzotriazole, N-hydroxysuccinamide, N-hydroxy-5-norbomane-2,3-dicarboxyimide, and the like) method, and Woodward's method. The present protein obtained by chemical synthesis can be suitably purified in accordance with various kinds of conventional purification methods as described above.

Proteins shown by partial sequences of the proteins according to the present invention can also be obtained by cleaving the proteins according to the present invention, by a suitable peptidase.

### (Antibody)

A further aspect of the present invention relates to antibodies recognizing the proteins according to the present invention. The antibodies can be prepared using the present proteins as antigens. The antigens to be used may be the present proteins or fragments thereof The antigen consist of amino acids of at least eight, preferably at least ten, more preferably at least twelve, and even more preferably fifteen or more amino acids. In order to prepare specific antibodies to the present proteins, it is preferable to use a region comprising a characteristic amino acid sequence of the present proteins. The amino acid sequence of this region is not necessarily required to be identical to an amino acid sequence of the present proteins or fragments thereof. An amino acid sequence of a site that is exposed outward on a tertiary structure thereof, may be preferable for such an amino acid sequence. Even if the amino acid sequence of the exposure site is not continuous on the primary structure, it is sufficient for the amino acid sequence to be continuous with respect to the exposure site. The antibodies are not particularly limited, and can be any antibody, as long as it can specifically recognize the present proteins. The phrase "specifically recognize the present proteins" means, for example, to bind to the present proteins, but not bind to or weakly bind to other proteins than the present proteins. The presence or absence of the recognition can be determined by known antigen-antibody binding reactions.

The antibodies can be produced by utilizing known antibody producing methods. For example, the antibodies can be obtained by administering to an animal an antigen alone, or an antigen bound to a carrier, with or without an adjuvant, and thereby inducing immunity, such as a humoral response, and/or a cellular response. Any known carrier can be used, as long as it does not exhibit an adverse action against the host, and is capable of enhancing the antigenicity of the antigen. Specifically, examples of the carrier include cellulose, polymeric amino acids, albumin, and keyhole limpet hemocyanin. Examples of the adjuvant include Freund's complete adjuvant (FCA), Freund's incomplete adjuvant (FIA), Ribi (MPL), Ribi (TDM), Ribi (MPL + TDM), Bordetella pertussis vaccine, muramyldipeptide (MDP), aluminium adjuvant (ALUM), and combinations of these. Mouse, rat, rabbit, goat, horse, or the like, can be preferably used as animals for immunization.

A polyclonal antibody can be acquired from the serum of an animal that was administered with an antigen by using any known method for recovering an antibody. Immunoaffinity chromatography may be employed as a preferable example of the method for recovering an antibody.

A monoclonal antibody can be produced utilizing a hybridoma that is prepared by collecting an antibody-producing cell, (for example, lymphocytes derived from spleen or lymph nodes,) from an animal that was administered with an antigen, and then fusing it with a well known immortalized cell (for example, myeloma strain such as P3-X63-Ag8 line). For example, the antibody-producing cell is fused with an immortalized cell by any known method to prepare a hybridoma which is subsequently subjected to cloning. The various cloned hybridomas are used to screen for a hybridoma that produces an antibody specifically recognizing a protein of the present invention. The antibody can be then recovered from a culture solution of that hybridoma.

A polyclonal antibody or monoclonal antibody, which is capable of recognizing or binding to a protein of the present invention, can be utilized as an antibody for purification of the present protein, reagent, labeling marker or the like. In particular, an antibody that inhibits the function of the present protein can be used for regulating the function of the present protein, and is useful for elucidating, preventing, improving, and/or treating various kinds of diseases due to an abnormality of a present protein, in amount and/or function.

### (Method of Identifying a Compound)

A still further aspect of the present invention relates to methods of identifying compounds that inhibit or enhance the function of the proteins according to the present invention, or compounds that inhibit the expression of the polynucleotides according to the present invention. Since it was found that the expression of the present polynucleotide is high in an esophageal cancer, such as an esophageal adenocarcinoma, the present inventors believe that prevention and/or treatment of these diseases can be conducted by inhibiting the function of the present proteins and/or inhibiting the expression of the present polynucleotides. Therefore, an identification method according to the present invention may be preferably a method of identifying a compound that inhibits the function of the present proteins, or compounds that inhibit the expression of the present polynucleotides.

The identification methods according to the present invention can be carried out using at least one member selected from the proteins, the polynucleotides, the recombinant vectors, the transformants, and the antibodies which are provided in the present invention, by employing any known pharmaceutical screening system. The present identification methods include any methods that are carried out in vitro or in vivo. The present identification methods allow screening for antagonists by drug design based on the structure of the present proteins, screening for an inhibitor of the expression at the gene level by utilizing a protein synthesis system, or screening for a substance recognized by an antibody by utilizing the antibody, or the like.

A method of identifying a compound that inhibits or enhances the function of the proteins according to the present invention can be carried out using an experimental system capable of measuring the function of the present protein, which comprises allowing the present protein coexist with a compound to be tested (test compound), under conditions allowing the interaction of the present protein with the test compound, and measuring the function of the present protein, subsequently, comparing the function of the present protein in the presence of the test compound to the function of the present protein in the absence of the test compound, and finally detecting the presence, the absence, or the change of the function of the present protein, such as reduction, enhancement, disappearance, and appearance. Where the function of the present protein is reduced or eliminated in the presence of a test compound, in comparison to the function of the present protein in the absence of the test compound, it can be determined that the test compound inhibits the function of the present protein. Oppositely, where the function of the present protein is enhanced or appeared in the presence of a test compound, it can be determined that the test compound enhances the function of the present protein. The function of the proteins can be measured by direct detection of the function, or by introducing a signal as an indicator of the function into an experimental system and detecting the signal. Examples of a signal include enzymes such as GST, tag peptides such as His-tag, Myc-tag, HA-tag, FLAG-tag, or Xpress-tag, or a fluorescent protein. Any other labeling substance can be used, as long as it is used in a conventional method of identifying a compound.

The function of the proteins according to the present invention may be, for example, preferably a GEF activity for a Rho family protein, more preferably a function of binding to a Rho family protein to exhibit a GEF activity. In other words, the function of the protein may be, for example, a function of activating a Rho family protein, more preferably a function of binding to a Rho family protein to activate the Rho family protein.

The proteins according to the present invention can be considered to bind to a Rho family protein to exhibit a GEF activity. Therefore, the present inventors believe that a GEF activity of the present proteins can be inhibited by inhibiting the binding of the present proteins to a Rho family protein. In addition, the present inventors believe that a GEF activity of the present proteins can be enhanced by increasing the binding of the present proteins to a Rho family protein. Methods of identifying compound that inhibit or increase the binding of the present proteins to a Rho protein, such as Cdc42, are included in the scope of the present identification method

A method of identifying a compound that inhibits or enhances a GEF activity of the proteins according to the present invention for a Rho family protein, can be carried out, for example, by allowing a present protein to co-exist with a Rho family protein, which may be activated by the present protein, and measuring the amount of an activated Rho family protein in the presence or absence of a test compound. In the case that the amount of an activated Rho family protein is reduced in the presence of a test compound, compared to in the absence of the test compound, it can be determined that the test compound inhibits a GEF activity of the proteins according to the present invention for a Rho family protein. Oppositely, in the case that the amount of an activated Rho family protein is increased in the presence of a test compound, compared to in the absence of the test compound, it can be determined that the test compound enhances a GEF activity of the protein according to the present invention for a Rho family protein. An activated Rho family protein can be measured by using an antibody raised against the protein. For example, an activated Rho family protein can be measured using an effector molecule capable of binding to the activated Rho family protein, but not binding or weakly binding to the non-activated Rho family protein. Specifically, as shown in Example 3, it is conducted to detect the binding of an activated Rho family protein, to a GST-fused effector molecule fragment containing a binding site to the activated Rho family protein, by pull-down assay. In such a method, an amount of the activated Rho family protein can be measured by electrophoresis and Western blotting. An effector molecule that binds to an activated Rho family protein is different depending on the type of a Rho family protein. Therefore, a suitable effector protein may be selected for use according to the kind of a Rho family protein used. For example, activated Cdc42 is known to bind to the effector molecule, PAK-1 (p21 activated kinase). Therefore, PAK1 may be a suitable effector molecule for use to identify a compound that inhibits or enhances a GEF activity of the present proteins for Cdc42.

A method of identifying a compound that inhibits or enhances a GEF activity of the proteins according to the present invention for a Rho family protein, can be also carried out by allowing the present proteins to co-exist with a Rho family protein, which may be activated by the present proteins and is bound to radioactive isotope-labeled GDP, and with GTP, and subsequently measuring the amount of an activated Rho family protein in the presence or absence of a test compound. The activated Rho family protein can be quantitatively determined by measuring the reduced amount of the Rho family protein being bound to radioactive isotope-labeled GDP. Such a method can be carried out by referring a method described in a known literature (Glaven J.A. et al., Journal of Biological Chemistry, 1996, Vol. 271, No. 44, p. 27374-27381).

The phrase "inhibiting a GEF activity for a Rho family protein" means to inhibit the conversion of a GDP-bound inactive form of Rho family protein to a GTP-bound active form of Rho family protein. More specifically, it means to reduce or to delete the amount of a GTP-bound active form of Rho family protein. The phrase "enhancing a GEF activity for a Rho family protein" means to enhance the conversion of a GDP-bound inactive form of Rho family protein to a GTP-bound active form of Rho family protein. More specifically, it means to increase the amount of a GTP-bound active form of Rho family protein.

A Rho family protein used in the identification method according to the present invention may be a protein lacking of a part thereof, or a protein labeled with a labeling substance, as long as the activation thereof by a protein according to the present invention is not affected. The labeling substance is exemplified in the above description.

A method of identifying a compound that inhibits or enhances the binding of the proteins according to the present invention to a Rho family protein can be carried out, for example, by expressing the present proteins using gene manipulation techniques to obtain the proteins, and detecting the binding of the proteins to a Rho family protein in the presence or absence of a test compound Specifically, for example, the present proteins can be subjected to a reaction in the presence or absence of a test compound, with a Rho family protein that is expressed as a GST-fusion protein by using gene manipulation techniques followed by binding to a glutathione-Sepharose. The identification of a compound that inhibits or enhances the binding of the present proteins to a Rho family protein can be achieved by measuring the present proteins that bind to the Rho family protein bound to a glutathione-Sepharose. Where the binding of both proteins is reduced or eliminated, in the presence of a test compound, in comparison to the binding of both proteins in the absence of the test compound, it can be determined that the test compound inhibits the binding of the present proteins to the Rho family proteins. Oppositely, where the binding of both proteins is increased in the presence of a test compound, it can be determined that the test compound enhances the binding of the present protein to the Rho family protein. The quantitative measurement of the present proteins can be carried out, for example, by using antibodies according to the present invention. An antibody labeled with a labeling substance such as enzymes (e.g. HRP or ALP), a radioactive isotope, a fluorescent substance, or biotin may be used. Alternatively, a labeled second antibody may be used. In the case of using the present proteins fused with a tag-peptide, the quantitative measurement thereof can be carried out using an antibody against the tag-peptide. Alternatively, the present proteins may be used after labeling directly with a labeling substance, such as the aforementioned enzyme, radioactive isotope, fluorescent substance, biotin, or the like. In such a case, the quantitative measurement can be carried out by measuring the labeling substance.

More specifically, a method of identifying a compound that inhibits or enhances the binding of the proteins according to the present invention to a Rho family protein, can be carried out using suitable cells in which a polynucleotide encoding the present protein are co-expressed with a polynucleotide encoding a Rho family protein, and using an experimental system which detects the binding of both proteins, by pull-down assay.

A well known two-hybrid method can be used for carrying out a method of identifying a compound that inhibits or enhances the binding of the protein according to the present invention to a Rho family protein. For example, the method can be carried out wherein a plasmid for expressing a fusion protein of the protein according to the present invention and a DNA binding protein, a plasmid for expressing a fusion protein of a Rho family protein and a transcription activating protein, and a plasmid containing a reporter gene that is linked to a suitable promoter gene, are introduced to a yeast, a eukaryotic cell, or the like. The identification of a compound that inhibits or enhances the binding of the present protein to a Rho family protein can be achieved by comparing the amount of expression of the reporter gene, in the presence of a test compound, to an amount of expression of the reporter gene in the absence of the test compound. In the case that the amount of expression of the reporter gene in the presence of the test compound is reduced or eliminated, compared to the amount of expression of the reporter gene in the absence of the test compound, it can be determined that the test compound inhibits the binding of the present protein to a Rho family protein. Oppositely, in the case that the amount of expression of the reporter gene in the presence of a test compound is increased, it can be determined that the test compound enhances the binding of the present protein to the Rho family protein. Any reporter genes that are used in a conventional reporter assay can be used herein. A reporter gene can be exemplified by a gene having an enzyme activity, such as, luciferase, β-Gal, chloramphenicol acetyl transferase, or the like. The expression of the reporter gene can be detected by determining the activity of the gene product, for example, an enzyme activity in the case of using the reporter gene exemplified in the above.

A surface plasmon resonance sensor, such as, the BIACORE system, or the like, can also be used in the method of identifying a compound that inhibits or enhances the binding of the protein of the present invention to a Rho family protein. Alternatively, Scintillation proximity assay (SPA), or a method employing fluorescence resonance energy transfer (FRET), can also be used for carrying out the present identification method.

A method of identifying a compound that inhibits or enhances the expression of a polynucleotide according to the present invention can be carried out using an experimental system capable of measuring the expression of the present polynucleotide, which comprises allowing the present polynucleotide co-exist with a test compound under conditions allowing the present polynucleotide with the test compound, and measuring the expression of the present polynucleotide. Subsequently, the expression of the present polynucleotide in the presence of the test compound can be compared to the expression of the present polynucleotide in the absence of the test compound, for detecting the presence, the absence, or the change of the expression of the present polynucleotide, such as reduction, enhancement, disappearance, and appearance. In the case that the expression of the present polynucleotide is reduced or disappeared in the presence of a test compound in comparison to the expression of the present polynucleotide in the absence of the test compound, it can be determined that the test compound inhibits the expression of the present polynucleotide. Oppositely, in the case that the expression of the present polynucleotide is increased in the presence of a test compound, it can be determined that the test compound enhances the expression of the present polynucleotide.

Specifically, for example, the method of identifying a compound that inhibits or enhances the expression of the polynucleotide according to the present invention, can be carried out using an experimental system using the transformant according to the present invention, for expressing the present polynucleotide, which comprises contacting the transformant with a test compound, and then measuring the expression of the present polynucleotide. The expression of the present polynucleotide can be measured easily by detecting the amount of expressed protein, or by detecting the function of the protein, for example, a GEF activity for a Rho family protein. Further, the expression of the present polynucleotide can be measured also by introducing, for example, a signal as an indicator of the expression into the experimental system, and detecting the signal. Examples of a signal include: enzymes such as GST; tag peptides such as His-tag, Myc-tag, HA-tag, FLAG-tag, or Xpress-tag; or a fluorescent substance. A method of detecting these signals is well known to those skilled in the art.

A method of identifying a compound that inhibits or enhances the expression of the polynucleotide according to the present invention can also be carried out by, for example, preparing a vector that comprises a promoter region of a gene corresponding to the present polynucleotide and a reporter gene linked downstream of the promoter region instead of the present polynucleotide, and contacting a cell, e.g. a eukaryotic cell, which contains the vector, with the test compound, and then determining the presence or absence of, or a change in expression of the reporter gene. Any reporter genes that are used in a conventional reporter assay can be used herein. Specifically, a reporter gene can be exemplified by a gene having an enzyme activity, such as, luciferase, β-Gal, chloramphenicol acetyl transferase, or the like. The expression of the reporter gene can be detected by determining the activity of the gene product, for example, an enzyme activity in the case of using a reporter gene exemplified above.

### (Compound)

A compound obtained by an identification method according to the present invention can be utilized as a candidate compound for an inhibitor of the expression of a polynucleotide according to the present invention, an inhibitor or an antagonist of the function of a protein according to the present invention, For example, the compound can be utilized as a candidate compound for an inhibitor of a GEF activity for a Rho family protein, namely, an inhibitor of an activation of a Rho family protein. A compound obtained by the present identification method can be utilized as a candidate compound for an agent for enhancing the expression of the polynucleotide according to the present invention, or an agent for enhancing the function of the protein according to the present invention. Since it was found that the expression of the present polynucleotide is high in an esophageal cancer, such as an esophageal adenocarcinoma, the present inventors believe that prevention and/or treatment of the diseases can be conducted by inhibiting the function of the present protein and/or inhibiting the expression of the present polynucleotide. Therefore, a compound obtained by the identification method according to the present invention may be preferably a compound that inhibits the function of the present protein and/or a compound that inhibits the expression of the present polynucleotide. These candidate compounds can be prepared as a medicament by taking into consideration the balance between usefulness and toxicity. Such a medicament is useful for preventing and/or treating various kinds of symptoms due to an abnormality in the function of the present protein and/or an abnormality in the expression of the present polynucleotide. The compounds according to the present invention include compounds that are obtained by other methods than the present identification methods if they are capable of inhibiting the function of the present protein and/or the expression of the present polynucleotide, or are capable of enhancing the function of the present protein and/or the expression of the present polynucleotide.

### (Pharmaceutical Composition)

A further aspect of the present invention relates to a medicament or a pharmaceutical composition which is based on inhibiting or antagonizing the function of a present protein and/or the expression of a present polynucleotide, or which is based on enhancing the function of the present protein and/or the expression of the present polynucleotide. The medicament or the pharmaceutical composition contains a protein, a polypeptide, a recombinant vector, a transformant, an antibody, or a compound, which is provided according to the present invention, as an active ingredient.

A medicament according to the present invention can be a medicament that contains an effective amount of at least one member selected from a protein, a polynucleotide, a recombinant vector, a transformant, an antibody, or the compound, which is provided according to the present invention, as an active ingredient. In general, it is preferable to prepare a pharmaceutical composition using one or more kinds of pharmaceutically acceptable carriers (pharmaceutical carriers).

An amount of the active ingredient contained in the pharmaceutical composition according to the present invention can be suitably selected from a wide range. In general, a suitable amount may fall within a range of approximately 0.00001 to 70 wt%, preferably approximately 0.0001 to 5 wt%.

A pharmaceutical carrier may be a diluent or excipient, which can be generally used in accordance with the form of use of the pharmaceutical composition, such as, a filler, an extender, a binder, a wetting agent, a disintegrator, and/or a lubricant. These can be suitably selected and used in accordance with the form of use of the pharmaceutical composition used.

The pharmaceutical carrier may be, for example, water, a pharmaceutically acceptable organic solvent, collagen, polyvinyl alcohol, polyvinylpyrrolidone, carboxyvinyl polymer, sodium alginate, soluble dextran, sodium carboxymethyl starch, pectin, xanthan gum, acacia gum, casein, gelatin, agar, glycerin, propylene glycol, polyethylene glycol, vaseline, paraffin, stearyl alcohol, stearic acid, human serum albumin, mannitol, sorbitol and lactose. One or a combination of two or more kinds of these carriers may be suitably selected, and used in accordance with the form of use of a pharmaceutical composition of the present invention.

As desired, various ingredients used in conventional protein preparations can be suitably used herein, such as, a stabilizer, a bacteriocide, a buffer agent, an isotonizing agent, a chelating agent, a pH adjuster, or a surfactant, for preparing the pharmaceutical composition.

As a stabilizer, the following may be used: human serum albumin, common L-amino acids, sugars, and cellulose derivatives. These can be used independently or in combination with a surfactant, and the like. Use of these in such a combination may give increased stability to an active ingredient. An L-amino acid is not particularly limited, and may be any one of glycine, cysteine, glutamic acid, and the like. A sugar is not particularly limited, and may be any one of the monosaccharides (such as glucose, mannose, galactose, and fiuctose), sugar alcohols (such as mannitol, inositol, and xylitol), disaccharides (such as sucrose, maltose, and lactose), polysaccharides (dextran, hydroxypropylstarch, chondroitin sulfate, and hyaluronic acid), derivatives thereof, and so on. A cellulose derivative is not particularly limited, and may be any one of methylcellulose, ethylcellulose, hydroxyethylcellulose, hydroxypropylcellulose, hydroxypropylmethylcellulose, sodium carboxymethylcellulose, and the like.

A surfactant is not particularly limited, and can be both an ionic surfactant and/or a non-ionic surfactant. As a surfactant, the following may be used: polyoxyethyleneglycol sorbitan alkyl ester base; polyoxyethylene alkyl ether base; sorbitan monoacyl ester base; or a fatty acid glyceride base.

As a buffer agent, the following may be used: boric acid; phosphoric acid; acetic acid; citric acid; ∈-aminocaproic acid; glutamic acid; and/or a salt thereof, for example, an alkali metal salt and/or an alkaline earth metal salt, such as a sodium salt, a potassium salt, a calcium salt and a magnesium salt.

As an isotonizing agent, the following may be used: sodium chloride; potassium chloride; sugars; or glycerin.

As a chelating agent, sodium edentate and citric acid may be used.

The medicaments and the pharmaceutical compositions according to the present invention can be used as solution preparations. Alternatively, they can be freeze-dried, so as to be in a good state for preservation. They can be used by dissolving them in water, a buffered solution containing saline, and the like, and then adjusting them to a suitable concentration, at the time of use.

The medicaments and the pharmaceutical compositions according to the present invention can be used as agents for preventing and/or treating a disease due to an abnormality in the function of a present protein and/or an abnormality in the expression of a present polynucleotide. In addition, the medicaments and the pharmaceutical compositions can be used in methods for preventing and/or treating the disease.

For abnormal symptoms due to an excess of a function of a protein according to the present invention, and/or the expression of a polynucleotide according to the present invention, an effective amount of an inhibitor that inhibits the function of a present protein and/or the expression of a present polynucleotide, may be administered to a subject together with a pharmaceutically acceptable carrier. The administration may result in obtaining an effect such as prevention, improvement, or treatment of the abnormal symptoms. Alternatively, the similar effect can be obtained by inhibiting the intrinsic expression of a present polynucleotide using an expression block method. The inhibition of the expression of a present polynucleotide can be achieved, for example, by using an anti-sense oligonucleotide, such as an oligonucleotide consisting of a partial sequence of a present polynucleotide. An oligonucleotide corresponding to a non-coding region of a present polynucleotide as well as an oligonucleotide corresponding to a coding region thereof, is useful as an anti-sense oligonucleotide used herein. In order to specifically inhibit the expression of a present polynucleotide, it is preferable to use a nucleotide sequence of a characteristic region of the polynucleotide.

A disease due to an abnormality in the function of a present protein and/or an abnormality in the expression of a present polynucleotide may be, for example, a cancer disease preferably exemplified by an esophageal adenocarcinoma. The tissue distribution of the polynucleotide shown by the nucleotide sequence set forth in SEQ ID NO: 1, which is a specific example of a polynucleotide according to the present invention, was found to be 2.78 times higher in an esophageal adenocarcinoma that is one of esophagus cancers, as compared to that in a normal esophagus tissue. The tissue distribution was examined using BioExpress (GeneLogic), disease database information. As described above, the protein encoded by the polynucleotide shown by the nucleotide sequence set forth in SEQ ID NO: 1 has a GEF activity. Therefore, the protein can be considered to work as Rho-GEF. Among the Rho-GEF genes isolated so far, the following genes are known to relate to cancer: vav (Non-Patent References 3 and 4); ost (Non-Patent Reference 5); ibc (Non-Patent Reference 6) and the like. Thus, the present inventors believe that the high expression of the present polynucleotides relates to a cancer disease, for example, an esophageal cancer, such as an esophageal adenocarcinoma, and the like. Further, the present inventors believe that the prevention and/or treatment of these diseases can be conducted by inhibiting the function of the present proteins and/or the expression of the present polynucleotides. The medicaments and the pharmaceutical compositions according to the present invention may be useful as agents for preventing and/or treating an esophageal cancer. In addition, the medicaments and the pharmaceutical compositions may be used in methods of preventing and/or treating an esophageal cancer.

Suitable dosage ranges of the medicament and the pharmaceutical composition according to the present invention are not particularly limited, and can be determined in accordance with the following: effectiveness of the ingredients contained therein; the administration form; the route of administration; the type of disease; the characteristics of the subject (e.g., body weight, age, symptomatic conditions, and whether a subject is taking other pharmaceutical agents); and the judgment of a physician in charge. In general, a suitable dosage may fall, for example, within a range of about 0.01 µg to 100 mg, per 1 kg of the body weight of the subject, and preferably within a range of about 0.1 µg to 1 mg, per 1 kg of body weight. However, the dosage may be altered using conventional experiments for optimization of a dosage that are well known in the art. The aforementioned dosage can be divided for administration once to several times a day Alternatively, periodic administration once every few days or few weeks can be employed.

When administering the medicament or the pharmaceutical composition according to the present invention, the medicament or the pharmaceutical composition may be used alone, or may be used together with other compounds or medicaments useful for preventing and/or treating the target disease.

In terms of a route of administration, it may be either systemic administration or local administration. The route of administration that is appropriate for a particular disease, symptomatic condition, or other factors, should be selected. For example, parenteral administration including normal intravenous injection, intra-arterial administration, subcutaneous administration, intracutaneous administration, and intramuscular administration can be employed. Oral administration can be also employed. Further, transmucosal administration or dermal administration can be employed. In the case of use for cancer disease, it may be preferable to employ a direct administration into the tumor by injection, and the like.

In terms of an administration form, various forms can be selected in accordance with a treatment purpose. For example, a solid formulation may be employed such as a tablet, pill, powder, powdered drug, fine granule, granule, or a capsule. Alternatively, a liquid formulation can be employed such as an aqueous formulation, ethanol formulation, suspension, fat emulsion, liposome formulation, clathrate such as cyclodextrin, syrup, or an elixir. These can be further classified, according to the administration route, into an oral formulation, parenteral formulation (drip injection formulation or injection formulation), nasal formulation, inhalant formulation, transvaginal formulation, suppositorial formulation, sublingual agents, eye drop formulation, ear drop formulation, ointment formulation, cream formulation, transdermal absorption formulation, transmucosal absorption formulation, and the like, which can be respectively blended, formed and prepared according to conventional methods.

### (Diagnosing Method)

The proteins, polynucleotides, recombinant vectors, transformants, antibodies, or the compounds, which are provided in the present invention, can be used by themselves as a means for diagnosing a disease, such as a diagnostic marker or a diagnostic reagent.

According to the present invention, for example, use of all or a part of a polynucleotide according to the present invention allows the specific detection of the presence or absence of an abnormality in a polynucleotide or a gene containing the polynucleotide, or the presence or absence of expression thereof, in an individual, or in various kinds of tissues. The detection of a polynucleotide according to the present invention allows for a diagnosis of susceptibility to, onset of, and/or prognosis of, a disease due to an abnormality in the amount of a polynucleotide or a gene containing the polynucleotide, and/or, an abnormality in the function thereof

Diagnosis of a disease can be carried out, for example, by detecting the presence of a polynucleotide according to the present invention, by determining the existing amount thereof, and/or by identifying a mutation, with respect to a sample to be tested (test sample). In comparison to a normal control sample, a change in the existence of a present polynucleotide, and a quantitative change thereof, can be detected. Alternatively, an amplified product obtained by amplifying a present polynucleotide using a known method may be subjected, for example, to the measurement of a change in size. In comparison to a normal genotype, a mutation such as deletion or insertion can be detected. Further, a polynucleotide amplified from a test sample may be subjected, for example, to hybridization to a labeled polynucleotide according to the present invention, which allows the isolation of a point mutation. The detection of such a mutation allows the aforementioned diagnosis.

The present invention can provide a qualitative or quantitative measurement method, for a polynucleotide according to the present invention, in a test sample. Further, a qualitative or quantitative measurement method for the mutation, in the specific region of a polynucleotide, can also be provided.

The tissue distribution of the polynucleotide shown by the nucleotide sequence set forth in SEQ ID NO: 1, was found to be 2.78 times higher in an esophageal adenocarcinoma that is one of esophageal cancers, compared to that in a normal esophageal tissue. Meanwhile, as described in the above, the present inventors considered that the high expression of a present polynucleotide relates to an esophageal cancer. Therefore, the detection of the increased amount of expression of the polynucleotide in a test sample allows execution of a method of determining whether the test sample is a test sample that is derived from an esophageal cancer or not. Such a determination method may also be included in the scope of the present invention. In this determination method, the increased amount of expression of the polynucleotide can be detected by comparing a test sample to a normal control sample. A human esophagus-derived tissue may be preferably used as a test sample. A normal human esophagus-derived tissue may be preferably used as a control sample. In the case that the amount of expression of the polynucleotide is increased compared to that in a control sample, preferably at least 2.5 times or more, more preferably at least 3 times or more, it can be determined that the test sample is a human esophageal cancer-derived sample. The polynucleotides according to the present invention other than the polynucleotide shown by the nucleotide sequence set forth in SEQ ID NO: 1 can also be used for carrying out this determination method. The polynucleotide shown by the nucleotide sequence set forth in SEQ ID NO: 3 can be used for such a polynucleotide. The phrase "amount of expression of the polynucleotide according to the present invention" means the amount of the transcription product of the polynucleotide.

A test sample is not limited to an esophagus-derived tissue. Any tissues and cells derived from a living body can be used. Specifically, a sample derived from a living organism such as a cell, blood, urine, saliva, spinal fluid, biopsy tissue, or autopsy material, and the like, may be used as a test sample. As desired, a nucleic acid may be extracted from a test sample to prepare a nucleic acid sample for use. A nucleic acid may be any of a genomic DNA, RNA, and cDNA. A nucleic acid also may be enzymatically amplified by employing PCR, or other amplification methods. A nucleic acid sample may also be prepared according to various methods, for facilitating detection of a target sequence, for example, denaturation, digestion with restriction enzymes, electrophoresis, or dot blotting.

Any known gene detection methods can be used for detecting a polynucleotide according to the present invention or a gene containing the polynucleotide. Specifically, for example, plaque hybridization, colony hybridization, Southern blotting, Northern blotting, the NASBA method, reverse transcription-polymerase chain reaction (RT-PCR), or the like can be used. In addition, a gene detection method which allows cell level measurement, such as in situ RT-PCR, in situ hybridization, or the like, can be used for the detection. In such a gene detection method, it is useful to use an oligonucleotide, which consists of a partial sequence of a present polynucleotide, and has the property as a probe or a primer, for carrying out the isolation and/or the amplification of the polynucleotide, a gene containing the polynucleotide, or a mutant gene thereof. The phrase "oligonucleotide having the property as a probe" means an oligonucleotide that is capable of specifically hybridizing only to the present polynucleotide, and consists of a characteristic sequence of a present polynucleotide. The phrase "oligonucleotide having the property as a primer" means an oligonucleotide that is capable of specifically amplifying only a present polynucleotide, and consists of a characteristic sequence of a present polynucleotide. Further, when detecting a mutant gene capable of being amplified, a primer or a probe having a sequence with a predetermined length, which contains a mutation site within the gene, is prepared and used. A probe and a primer may have a nucleotide sequence consisting of, preferably from about 5 to 50 nucleotides, more preferably from about 10 to 35 nucleotides, and even more preferably from about 15 to 30 nucleotides. Specifically, an oligonucleotide shown by the nucleotide sequence set forth in SEQ ID NO: 7, SEQ ID NO: 8, or SEQ ID NO: 9 can be preferably used as a primer for amplifying a polynucleotide of the present invention or a fragment thereof, or as a probe for detecting a present polynucleotide. A labeled probe is normally used as the probe, but the unlabeled prove can also be used. Alternatively, the detection can also be carried out by measuring the specific binding to a ligand that was labeled directly or indirectly. Various methods are known for labeling a probe and a ligand. For example, nick translation, random priming, or a method utilizing kinase treatment, may be used. Labeling substances suitable for use include a radioactive isotope, biotin, a fluorescent substance, a chemiluminescent substance, an enzyme, an antibody, and the like.

PCR is preferable as a gene detection method, from the viewpoint of sensitivity Any well-known method of PCR can be employed, as long as it is a method that uses a primer capable of specifically amplifying a polynucleotide according to the present invention, a gene containing the polynucleotide, or a mutant gene thereof. For example, RT-PCR may be employed. In addition, various modified PCT methods used in the art can be applied.

In addition to detection of a gene, PCR allows quantitative measurement of a polynucleotide according to the present invention, a gene containing the polynucleotide, or a mutant gene thereof. Such an assay method may be exemplified by a competitive assay, such as, an MSSA method, or PCR-SSCP, which is known as a mutation detection method that utilizes a change in mobility accompanying a structural change of a single-stranded DNA.

According to the present invention, for example, use of a protein according to the present invention allows the specific detection of, the presence or absence of, an abnormality in the protein itself, and in its function, in an individual or in various kinds of tissues. The detection of an abnormality in a protein according to the present invention, and in its function, allows a diagnosis of susceptibility to, onset of, and/or prognosis of, a disease due to an abnormality in the amount of the protein and/or an abnormality in its function.

The diagnosis of a disease by detecting a protein can be carried out, for example, by detecting the presence of a protein, by determining the existing amount thereof, and/or by identifying a mutation, with respect to a sample to be tested (test sample). That is to say, a protein according to the present invention, and/or its mutant, may be quantitatively or qualitatively measured. In comparison to a normal control sample, a change in the existence of a present protein, and a quantitative change thereof, can be detected. Alternatively, in comparison to a normal control sample, a mutation can be detected, for example, by determining an amino acid sequence. The detection of such a change or a mutation allows the aforementioned diagnosis. A test sample is not limited so long as the sample has the present protein and/or its mutant. A biological sample derived from a living organism, such as blood, serum, urine, biopsy tissue, and the like, may be used as a test sample.

A protein according to the present invention, and the protein with a mutation, can be measured using a protein according to the present invention, a fragment thereof, or an antibody against the protein or the fragment. Specifically, for example, the protein shown by the amino acid sequence set forth in SEQ ID NO: 2, SEQ ID NO: 4, or SEQ ID NO: 6, a protein shown by the amino acid sequence having a deletion, substitution, insertion, or addition of one or several or more amino acids, in the amino acid sequence of the protein, the fragment thereof, or an antibody against the protein or the fragment, can be used.

Any protein detection methods, or protein quantitation methods which are well known in the art, can be used for quantitative or qualitative measurement of the protein. For example, the amino acid sequence analysis of a present protein allows a detection of a mutant protein. More preferably, an antibody (a polyclonal antibody or a monoclonal antibody) may be used for detecting the difference in the protein sequence, or the presence or absence of the protein.

The present invention includes a qualitative or quantitative measurement method for a present protein in a test sample, or a qualitative or quantitative measurement method for a mutation in the specific region of the protein.

Specifically, the aforementioned detection may be carried out by subjecting a test sample to immunoprecipitation, using a specific antibody raised against a present protein, and then analyzing the present protein by Western blotting or immunoblotting. Further, the detection of a present protein in a paraffin tissue section, or a frozen tissue section, may be carried out by means of immuno-histochemical techniques using a specific antibody raised against the present protein.

The preferable methods of detecting a present protein or its mutant, may be, for example, enzyme-linked immunosorvent assay (ELISA), radio immuno assay (RIA), immunoradiometric assay (IRMA), and immunoenzymometric assay (IEMA), including a sandwich method using a monoclonal antibody and/or a polyclonal antibody. Alternatively, radio immuno assay, competitive binding assay and the like may be employed.

A protein, polynucleotide, recombinant vector, transformant, and/or antibody, which are provided according to the present invention, can each be used by themselves or in combination as a reagent, or the like. The reagent may be contained in a substance such as a buffer solution, a salt, a stabilizer, and/or an antiseptic agent, in addition to at least one member selected from a protein, a polynucleotide, a recombinant vector, a transformant, and an antibody which are provided according to the present invention. A known formulation means may be introduced, in accordance with the respective properties, at the time of formulation. The reagent can be used, for example, in a determination method, a method of identifying a compound, or a method of measuring the present protein, or a present polynucleotide, which is provided according to the present invention. In addition, the reagent is useful, for example, in elucidating an intracellular signal transduction pathway wherein a protein or a polynucleotide according to the present invention may be involved and the reagent can be used in fundamental research, such as research for a disease due to an abnormality of the protein or the polynucleotide.

The present invention further provides a reagent kit containing at least one member selected from a protein, a polynucleotide, a recombinant vector, a transformant, and an antibody which are provided according to the present invention. The kit may further contain a substance necessary for carrying out a measurement, such as a labeling substance, for detecting a protein or a polynucleotide according to the present invention, an agent for detecting the labeling substance, a reaction diluent, a standard antibody, a buffer solution, a washing agent, and a reaction terminating solution. As a labeling substance, the proteins described above, radioactive isotype, or the like, can be used. A labeling substance may be previously linked to the protein or the polynucleotide according to the present invention. The present reagent kit can be used, for example, in the determination method, the method of identifying a compound, or the method of measuring a present protein, or a present polynucleotide, which is provided according to the present invention. In addition, the present reagent kit can also be used as a test agent or a test kit in the test method using the aforementioned measurement method. The present reagent kit can further be used as a diagnostic agent, or a diagnostic kit in the diagnostic method using the aforementioned measurement method.

Hereinafter, the present invention may be explained more specifically with the following examples. However, the aspect of the present invention is not limited to these examples.

### Example 1

### (Gene Cloning)

A human spleen-derived cDNA library was constructed in accordance with a method of Ohara et al. (Ohara, O. et al., DNA research, 1997, Vol. 4, p. 53-59). Specifically, double strand cDNA was synthesized by using a Superscript II reverse transcriptase kit (GIBCO BRL). At that time, an oligonucleotide (GACTAGTTCTAGATCGCGAGCGGCCGCCC(T)15: SEQ ID NO: 15, GIBCO BRL), which has a NotI site, was used as a primer, and a human spleen mRNA (Clontech: catalogue number 6542-1) was used as a template. The cDNA was ligated to an adaptor (GIBCO BRL) which has a SalI site, and then digested with NotI, followed by electrophoresis with 1 % concentration of a low melting point agarose to purify DNA fragments of 3 kb or more. The purified cDNA fragment was ligated with pBluescriptII SK+ plasmid treated with SalI-NoI restriction enzyme. The recombinant plasmid was introduced into E. coli. ElectroMax DH10B strain (GIBCO BRL) by electroporation. Approximately 10,000 recombinants were selected from the constructed cDNA library to determine the both terminal DNA sequences of these clones. Among them, approximately 420 clones, which contain novel genes, were selected to determine for the entire nucleotide sequences of the cDNAs. Sequencing was carried out using DNA sequencer (RISA: SHIMADZU) and a reaction kit purchased from PE Applied Biosystem. The most of the sequences of shotgun clones was determined by using a dye terminator method (terminator labeling method).

The ORF was predicted for cDNA clones, whose entire nucleotide sequences were determined, by a conventional analysis method using a computer program. Subsequently, the ORF region was analyzed by a domain motif search, to identify a cDNA containing a region encoding a DH/PH domain that is an active domain of Rho-GEF.

As a result, cDNA having a novel nucleotide sequence, and containing a DH/PH domain coding region, was identified. Hereinafter, the cDNA is referred to as sh04009.

In order to determine whether the identified cDNA clone sh04009 has a nucleotide sequence that encodes a full-length protein, *in silico* analysis was carried out using EST (Expression Sequence Tag) database. As a result, BU599407 was found to partially overlap with the 5'-terminal sequence of sh04009. BU599407 was expected to be a 5'-side sequence upstream of sh04009. Then, RT-PCR cloning was carried out for mRNAs to determine the existence of the sh04009 coding region (CDS) containing the 5'-side sequence. Specifically, cDNAs were produced by reverse transcription reaction from a human spleen derived polyA (+) RNA (Clontech) that was used as a template. The primers used were an oligonucleotide (SEQ ID NO: 7) containing the nucleotide sequence from the 1582^{nd} nucleotide to the 1554^{th} nucleotide of BU599407, and an oligonucleotide (SEQ ID NO: 8) containing the nucleotide sequence from the 100^{th} nucleotide to the 122^{nd} nucleotide of sh04009. The sh04009 CDS having a BU599407 sequence in its 5'-upstream, was obtained by PCR using pfu turbo (STRATAGENE) as a DNA polymerase. The CDS was inserted into pENTR/SD/D-TOPO (Invitrogen) in a reaction using TOPO cloning system, to prepare an entry vector. Hereinafter, sh04009 having a BU599407 sequence 5'-upstream thereof, is referred to full-length sh04009 gene. Sequencing analysis confirmed that the *in silico* prediction provided a correct nucleotide sequence for the CDS of full-length sh04009 gene, and that the nucleotide sequence of the CDS was correctly inserted into the entry vector. Sequencing reaction was carried out using DYEnamic ET Terminator Cycle Sequencing Kit (Amersham Biosciences). Electroporation and analysis were carried out using ABI PRISM 377.

As a result of sequencing analysis, full-length sh04009 gene was found to consist of the nucleotide sequence set forth in SEQ ID NO: 1 (6018 bp long), and to encode the amino acid sequence set forth in SEQ ID NO: 2 (574 amino acids). Comparison of full-length sh04009 gene with sh04009 resulted in findings of a lack of 140 nucleotides 5' upstream of sh04009, and one nucleotide difference. However, it was observed that the one nucleotide difference did not cause amino acid substitution. Specifically, the 1566^{th} position of the nucleotide sequence of full-length sh04009 gene (SEQ ID NO: 1) is a thymine (T), while the corresponding position of sh04009 is a cytosine (C). Thus, full-length sh04009 gene has CCT, a proline codon, from the 1564^{th} nucleotide to the 1566^{th} nucleotide, while sh04009 has CCC, also a proline codon, in the corresponding positions. Namely, the one nucleotide difference did not cause amino acid substitution. Further, sequence comparison for the short consecutive sequence containing the position of one nucleotide difference, showed that the corresponding position of the genome sequence (NT_009799.11 Hs13_9956) to the 1566^{th} position of the nucleotide sequence set forth in SEQ ID NO: 1, was a thymine (T).

A DH domain coding region of full-length sh04009 gene corresponds to the region consisting of the nucleotides from the 496^{th} nucleotide to the 1032^{nd} nucleotide of the nucleotide sequence set forth in SEQ ID NO: 1. A PH domain coding region corresponds to the region consisting of the nucleotides from the 1132^{nd} nucleotide to the 1449^{th} nucleotide of the nucleotide sequence set forth in SEQ ID NO: 1. In addition, a DH domain of the protein encoded by full-length sh04009 gene corresponds to 179 amino acid resides from the 166^{th} valine (Val) to the 344^{th} asparagine (Asn) of the amino acid sequence set forth in SEQ ID NO: 2. A PH domain corresponds to 106 amino acid resides from the 378^{th} leucine (Leu) to the 483^{rd} arginine (Arg) of the amino acid sequence set forth in SEQ ID NO: 2.

### Example 2

### (DNA expression and purification)

An expression vector for full-length sh04009 gene that was isolated in Example 1, was prepared using GATEWAY^{™} Cloning Technology (Invitrogen). Then, the expression vector was used for expressing a protein (hereinafter, may be referred to full-length sh04009 protein), which is encoded by the gene, as FLAG-tagged protein, in 293EBNA cells (Invitrogen). In addition, an expression vector for DNA (hereinafter, may be referred to sh04009DH/PH), which consists of a partial nucleotide sequence of the gene and contains a DH/PH domain coding region, was prepared in the same manner. Then, the expression vector was used for expressing a protein (hereinafter, may be referred to sh04009DH/PH protein), which consists of a partial amino acid sequence of a protein encoded by the gene and contains a DH/PH domain, as FLAG-tagged protein, in 293EBNA cells, as well. The expression was confirmed by Western blotting.

Specifically, a cDNA library was constructed from a human spleen derived polyA (+) RNA (Clontech) by reverse transcription reaction, for preparing an expression vector for full-length sh04009 gene and for expressing the gene. The cDNA library was used as a template for amplifying the ORF region, without a stop codon, of the gene. Amplification was carried out by using pfu turbo (STRATAGENE). Oligonucleotides shown by the respective nucleotide sequences set forth in SEQ ID NO: 7 and SEQ ID NO: 8, were used as amplification primers. Then, an amplification product was inserted into pENTR/SD/D-TOPO in a reaction using TOPO cloning system to prepare an entry vector. The prepared entry vector was used together with a C-terminal 3x FLAG-tagged protein-expression vector that was cleaved with BspEI, for preparing a full-length sh04009 gene expression plasmid, in a recombination reaction using LR clonase enzyme. Use of the expression plasmid allows production of C-terminal 3x FLAG-tagged full-length sh04009 protein. A sequencing analysis confirmed correct insertion of the nucleotide sequence of a coding region of full-length sh04009 gene into the expression plasmid. Sequencing reaction was carried out using DYEnamic ET Terminator Cycle Sequencing Kit (Amersham Biosciences). Electroporation and analysis were carried out using ABI PRISM 377.

Next, an expression vector for sh04009DH/PH was constructed using GATEWAY^{™} Cloning Technology (Invitrogen). Specifically, at first, the full-length sh04009 gene entry vector was used as a template for amplifying a polynucleotide of SEQ ID NO: 5. The polynucleotide of SEQ ID NO: 5 comprises a partial sequence consisting of the nucleotides from the 388^{th} nucleotide to the 1722^{nd} nucleotide of the nucleotide sequence set forth in SEQ ID NO: 1 (i.e. SEQ ID NO: 3), and an oligonucleotide of SEQ ID NO: 10 ligated to its 5'-terminal. The partial sequence contains a DH/PH coding region of full-length SH04009 gene. The oligonucleotide of SEQ ID NO: 10 consists of a kozak consensus sequence and a methionine codon. The amplification was carried out using pfu turbo (STRATAGENE), and using oligonucleotides shown by the respective nucleotide sequences set forth in SEQ ID NO: 9 and SEQ ID NO: 8 as primers. Then, an amplification product was inserted into pENTR/SD/D-TOPO in a reaction using TOPO cloning system to prepare an entry vector. The prepared entry vector was used together with a C-terminal 3x FLAG-tagged protein-expression vector that was cleaved with BspEI, for preparing a sh04009DH/PH expression plasmid, in a recombination reaction using LR clonase enzyme. Use of this expression plasmid allows production of a protein shown by a sequence of SEQ ID NO: 6 with FLAG-tag (3x) at the C-terminal. The sequence of SEQ ID NO: 6 is a sequence of SEQ ID NO: 4 with a methionine (Met) at the N-terminal. The sequence of SEQ ID NO: 4 consists of an amino acid residues from the 130^{th} to 574^{th} of the amino acid sequence set forth in SEQ ID NO: 2. Sequencing analysis confirmed correct insertion of the nucleotide sequence of a coding region of sh04009DH/PH. Sequencing reaction was carried out using DYEnamic ET Terminator Cycle Sequencing Kit (Amersham Biosciences). Electroporation and analysis were carried out using ABI PRISM 377.

The full-length sh04009 gene expression vector and the sh04009DH/PH expression vector were respectively transfected into 293EBNA cells by lipofection. The 293EBNA cells (6 x 10⁵ cells) had been seeded the day before transfection in T25 flask, and cultured in 5mL of culture medium (IMDM medium (SIGMA), 10 % fetal bovine serum, 4 mM glutamine, and 10 µg/mL gentamicin). On the next day, 3mL of culture medium was removed from the cultured cells. Then, the transfection was carried out by means of using Lipofectamine^{™} 2000 (Invitrogen). Specifically, at first, each expression vector was added to serum-free DMEM, subsequently mixed with DMEM containing Lipofectamine^{™} 2000, and incubated at room temperature for 20 minutes. The obtained mixture was added to 293EBNA cells that had been seeded the day before, and cultured at 37 °C in the presence of 5% CO₂. The cells subjected to transfection were cultured for more than 8 hours at 37 °C in the presence of 5% CO₂. After that, the cells were added with 3mL of culture medium for further culturing. On two days after transfection, the culture medium was removed from cultured cells to wash the cells with phosphate-buffered physiological saline (PBS). Then, the cells were lysed with lysis buffer A containing 1% protease inhibitor cocktail (SIGMA) to prepare a cell lysate. The lysis buffer A was composed of 25 mM Tris-HCl (pH 7.5),150 mM NaCl, 1 mM CaCl₂, and 1% Triton X-100.

Each cell lysate was mixed with an equal volume of SDS-PAGE sample buffer, and subjected to heat treatment at 100 °C for 5 minutes, to prepare a sample for electrophoresis. SDS-polyacrylamide gel electrophoresis (SDS-PAGE) was carried out, and then the gel was equilibrated for 5 minutes or more, in blotting buffer. The proteins were transferred onto PVDF membrane. After blotting, the PVDF membrane was subjected to blocking at 4 °C for overnight in a solution (TBS-T+BA) containing the TBS-T mixed with Block Ace (Dainippon Pharmaceutical) at a ratio of 3:1. After blocking, the PVDF membrane was washed with TBS-T for 10 minutes or more with shaking. As a SDS-PAGE sample buffer, β-ME Sample Treatment for Tris SDS (Daiichi Pure Chemical s) was used. The running buffer for SDS PAGE was composed of 100 mM Tris, 192 mM glycine, 0.1 % SDS, pH 8.3 (Bio Rad). The blotting buffer is composed of 25 mM Tris, 40 mM ∈-amino-n-caproic acid, 20% methanol, and 0.05% SDS. The TBS-T is composed of 150 mM NaCl, 10 mM Tris-HCl (pH 7.5), and 0.05% Tween-20.

Anti-FLAG M2 monoclonal antibody (SIGMA) that was diluted to 1000-fold with TBS-T+BA was added to the PVDF membrane, and incubated at 37 °C for 1 hour or more. After that, the PVDF membrane was washed three times with TBS-T with shaking (20 minutes or more for each wash), and HRP-labeled goat anti-mouse IgG antibody (Cell Signaling Technology), that was diluted to 1000-fold with TBS-T+BAwas added to the membrane. The membrane was incubated at 37 °C for 1 hour or more. Finally, the PVDF membrane was washed three times with TBS-T with shaking (20 minutes or more for each wash), followed by detection of the expressed protein that reacted with anti-FLAG antibody by using ECL Plus Western Blotting Detection System (Amersham biosciences). The chemiluminescence was visualized with detection device, Lumino Imaging Analyzer (TOYOBO).

The result is shown in Figure 1. Each of the cell lysates, that were prepared from the cells respectively transfected with the full-length sh04009 gene expression vector and the cells transfected with the sh04009DH/PH expression vector, showed a major band between 50 kDa and 100 kDa (lanes 2 and 3 in Figure 1, respectively). The deduced molecular weights of the full-length sh04009 protein and the sh04009DH/PH protein, which were expressed as FLAG-tagged proteins by these vectors, are approximately 72 kDa and approximately 58 kDa, respectively. On the other hand, none of these bands was detected in the cell lysate obtained in the same manner from cells that were added with Lipofectamine^{™} 2000 without transfecting with the vector (lane 1 in Figure 1). Therefore, the major bands observed in lanes 2 and 3 in Figure 1 are believed to be the full-length sh04009 protein and the sh04009DH/PH protein, respectively Thus, the full-length sh04009 protein and the sh04009DH/PH protein were obtained.

### Example 3

### (GEF activity of sh04009DH/PH protein for Cdc42)

The GEF activity of sh04009DH/PH protein was examined by effector pull-down assay using the sh04009DH/PH expression vector constructed in Example 2. The expression vector allows the expression of sh04009DH/PH protein as C-terminal 3x FLAG-tagged protein. Cdc42 was used as a Rho family protein. In order to express Cdc42, a Cdc42 gene expression vector was constructed using GATEWAY^{™} Cloning Technology (Invitrogen). Specifically, Cdc42 gene (SEQ ID NO: 11) was amplified by pfu turbo using spleen first strand DNA of Multiple Tissue cDNA Panels (Clontech) as a template. An amplified product was inserted into pENTR/D in a reaction using TOPO cloning system to prepare an entry vector. Oligonucleotides shown by the respective nucleotide sequences set forth in SEQ ID NO: 13 and SEQ ID NO: 14 were used as primers for amplifying Cdc42 gene. The constructed entry vector was then subjected to a recombination reaction in the presence of LR clonase enzyme, using an N-terminal 3x FLAG-tagged protein-expression vector that was cleaved with BspEI, to prepare a Cdc42 gene expression plasmid The expression plasmid allows the expression of Cdc42 (SEQ ID NO: 12) as an N-terminal 3x FLAG-tagged protein. Sequencing analysis confirmed correct insertion of the nucleotide sequence of a coding region of Cdc42 gene. Sequencing reaction was carried out using DYEnamic ET Terminator Cycle Sequencing Kit (Amersham Biosciences). Electrophoresis and analysis were performed using ABI PRISM 377.

The sh04009DH/PH expression vector and the Cdc42 gene expression vector were transfected into 293EBNA cells (5.0 x 10⁴ cells/well) that were plated in a 24 well plate. The transfection of cells with the vectors was carried out using Lipofectamine^{™} 2000. A cell transfected with an empty vector, which had no CDS that was inserted into the each vector, was used as a negative control. On two days after transfection, the culture medium was removed from cultured cells to wash the cells with PBS. Then, the cell was lysed with the lysis buffer B containing 1 % protease inhibitor cocktail (SIGMA) to prepare a cell lysate. Then, the cell lysate was subjected to a reaction with an effector bead (UPSTATE) at 4 °C for 1 hour. The effector bead used herein was a glutathione agarose conjugated with a GST-fusion protein that was prepared by adding a GST-tag to a binding domain of PAK-1 to an active Rho family protein. PAK-1 is known to bind to an active Cdc42 and the active Rac1. After the reaction, the effector bead was washed with the lysis buffer B, added with 40µL of a solution prepared by mixing the lysis buffer B with an equal volume of SDS-PAGE sample buffer, and then subjected to heat treatment at 100 °C for 5 minutes to prepare a sample. 8µL of the sample was applied for SDS polyacrylamide gel electrophoresis. The gel after electrophoresis was equilibrated for 5 minutes or more in tank blotting buffer. The proteins were transferred from the gel onto PVDF membrane. The PVDF membrane was subjected to blocking at 4 °C for overnight in TBS-T+BA. After blocking, the PVDF membrane was washed with TBS-T with shaking for 10 minutes or more. The lysis buffer B composed of 1 mM ethylene diamine tetra acetic acid (EDTA), 2 % glycerol, 1 % Triton X-100, 25 mM HEPES (pH 7.5),150 mM NaCl, and 10 mM MgCl₂. The tank blocking buffer was composed of 25 mM Tris, 192 mM glycine, and 20% methanol. The compositions of the SDS-sample buffer, running buffer, TBS-T and TBS-T+BA are the same as the buffers used in Example 2.

Detection of the FLAG-tagged protein was carried out with anti-FLAG-tag antibody. Specifically, anti-FLAG M2 monoclonal antibody (SIGMA) that was diluted to 1000-fold with TBS-T+BA was added to the PVDF membrane, and incubated at room temperature for 1 hour or more. After that, the PVDF membrane was washed three times with TBS-T with shaking (20 minutes or more for each wash), and HRP-labeled goat anti-mouse IgG antibody (Cell Signaling Technology) that was diluted to 1000-fold with TBS-T+BA was added to the membrane. The membrane was incubated at room temperature for 1 hour or more. Finally, the PVDF membrane was washed three times with TBS-T with shaking (20 minutes or more for each wash), followed by detection of the expressed protein that reacted with anti-FLAG antibody by using ECL Western Blotting Detection System (Amersham biosciences). The chemiluminescence was visualized with detection device, Lumino Imaging Analyzer (TOYOBO).

If the sh04009DH/PH protein had a GEF activity for Cdc42, the sh04009DH/PH protein would induce conversion of Cdc42 from an inactive form (GDP-bound form) to an active form (GTP-bound form). PAK-1 that was used as an effector bead is known to bind to an active Cdc42 and the active Rac1. Therefore, if the sh04009DH/PH protein had a GEF activity for Cdc42 and thereby activates Cdc42, Cdc42 that binds to the effector bead would increase in amount. Then, it was decided that the sh04009DH/PH protein had a GEF activity and activated Cdc42, when the band of Cdc42 was detected more strongly in the cell lysate prepared from the cells in which the sh04009DH/PH protein was co-expressed with Cdc42 than in the cell lysate prepared from the cells in which only the Cdc42 was expressed.

The results were shown in Figure 2. The upper panel of Figure 2 shows the results obtained by employing the effector pull-down assay for the aforementioned cell lysates. In the cell lysate (lane 4 in Figure 2) prepared from the cells in which the sh04009DH/PH protein was co-expressed with Cdc42, the band was detected more strongly that in the cell lysate (lane 2 in Figure 2) prepared from the cells in which only Cdc42 was expressed. That is to say, the active Cdc42, capable of binding to PAK-1, was increased in the cells in which Cdc42 was co-expressed with the sh04009DH/PH protein. The middle panel of Figure 2 shows the results of detection of Cdc42 contained in each cell lysate with anti-FLAG antibody. The amount of Cdc42 was almost the same in the cells in which only the Cdc42 was expressed, and in the cells in which Cdc42 was co-expressed with the sh04009DH/PH protein (lanes 2 and 4 in Figure 2). The lower panel of Figure 2 shows the results of detection of the sh04009DH/PH protein in each cell lysate with anti-FLAG antibody The amount of the sh04009DH/PH protein was almost the same in the cells in which only the sh04009DH/PH protein was expressed, and in the cells in which Cdc42 was co-expressed with the sh04009DH/PH protein (lanes 3 and 4 in Figure 2).

These results revealed that the sh04009DH/PH protein induced the conversion of Cdc42 from the inactive form to the active form. Thus, the present inventors believe that the sh04009DH/PH protein accelerates the GDP/GTP exchange reaction, and thereby converts Cdc42 from the inactive form to the active form. In other words, the present inventors believe that the sh04009DH/PH protein has a GEF activity.

The present inventors believe that the full-length sh04009 protein also has a GEF activity for Cdc42 as similar to the sh04009DH/PH protein, since it contains the sh04009DH/PH protein.

### INDUSTRIAL APPLICABILITY

The protein, encoded by a polynucleotide according to the present invention, exhibited a GEF activity for a Rho family protein, for example, for Cdc42. Use of the present proteins and polynucleotides allow elucidation and regulation of the Rho family protein mediated signal transduction pathway and cellular function. Further, use of the present proteins and polynucleotides allow for diagnosis, prevention and/or treatment of a disease due to an abnormal function of the present proteins and/or an abnormal expression of the present polynucleotides, for example, an esophageal cancer. Thus, the present invention is extremely useful in a wide field including basic research and pharmaceutical development.
GENERAL DESCRIPTION OF THE SEQUENCES
SEQ ID NO: 1: polynucleotide that encodes the protein (SEQ ID NO: 2) having a function as a guanine nucleotide exchange factor.
SEQ ID NO: 1: (496): (1032) a region encoding a Dbl homology domain.
SEQ ID NO: 1: (1132): (1449) a region encoding a pleckstrin homology domain.
SEQ ID NO: 3: polynucleotide containing a region encoding a Dbl homology domain and a pleckstrin homology domain, which is partial sequence of SEQ ID NO: 1 consisting of the nucleotides from the 388^{th} to the 1722^{nd}.
SEQ ID NO: 3: polynucleotide encoding the amino acid sequence set forth in SEQ ID NO: 4.
SEQ ID NO: 5: polynucleotide that has a kozak consensus sequence and a methionine codon in its 5'-terminal, followed by a partial sequence of SEQ ID NO: 1 consisting of the nucleotides from the 388^{th} to the 1722^{nd}, which contains a region encoding a Dbl homology domain and a pleckstrin homology domain.
SEQ ID NO: 5: polynucleotide encoding the amino acid sequence set forth in SEQ ID NO: 6.
SEQ ID NO: 5: (1): (4) a kozak consensus sequence.
SEQ ID NO: 5: (5): (7) a methionine codon.
SEQ ID NO: 7: designed oligonucleotide based on the sequence of SEQ ID NO: 1 for use as a primer.
SEQ ID NO: 8: designed oligonucleotide based on the sequence of SEQ ID NO: 1 for use as a primer.
SEQ ID NO: 9: designed oligonucleotide based on the sequence of SEQ ID NO: 1 for use as a primer.
SEQ ID NO: 10: designed oligonucleotide having a kozak consensus sequence followed by a methionine codon.
SEQ ID NO: 11: polynucleotide encoding Cdc42.
SEQ ID NO: 12: Cdc42
SEQ ID NO: 13: designed oligonucleotide based on the sequence of SEQ ID NO: 11 for use as a primer.
SEQ ID NO: 14: designed oligonucleotide based on the sequence of SEQ ID NO: 11 for use as a primer.
SEQ ID NO: 15: designed oligonucleotide for use as a primer.

## Claims

1. A polynucleotide shown by the nucleotide sequence set forth in SEQ ID NO: 1 in the sequence listing or by the complementary nucleotide sequence, or a polynucleotide encoding a protein shown by the amino acid sequence set forth in SEQ ID NO: 2 in the sequence listing, or a polynucleotide shown by the complementary nucleotide sequence of the polynucleotide.

2. A polynucleotide shown by the nucleotide sequence set forth in SEQ ID NO: 3 or SEQ ID NO: 5, in the sequence listing, or by the complementary nucleotide sequence, or a polynucleotide encoding a protein shown by the amino acid sequence set forth in SEQ ID NO: 4 or SEQ ID NO: 6 in the sequence listing, or a polynucleotide shown by the complementary nucleotide sequence of the polynucleotide.

3. A polynucleotide containing a polynucleotide shown by the nucleotide sequence set forth in SEQ ID NO: 3, in the sequence listing, or by the complementary nucleotide sequence, or a polynucleotide containing a polynucleotide encoding a protein shown by the amino acid sequence set forth in SEQ ID NO: 4, in the sequence listing, or a polynucleotide shown by the complementary nucleotide sequence of the polynucleotide, wherein the polynucleotide encodes a protein having a guanine nucleotide exchange factor activity (GEF) for Cdc42.

4. A polynucleotide selected from the following group, wherein the polynucleotide encodes a protein having a guanine nucleotide exchange factor (GEF) activity for Cdc42:
(i) a polynucleotide shown by a nucleotide sequence having a homology of at least approximately 70% with the nucleotide sequence of the polynucleotide according to claim 1, or claim 2,
(ii) a polynucleotide with a mutation or an induced mutation, such as deletion, substitution, or addition of one or several nucleotides in the nucleotide sequence of the polynucleotide according to claim 1 or claim 2, and
(iii) a polynucleotide that hybridizes to the polynucleotide according to claim 1 or claim 2 under stringent conditions.

5. A recombinant vector containing the polynucleotide according to any one of claims 1 to 4.

6. A transformant that has been transfected with the recombinant vector according to claim 5.

7. The transformant according to claim 6, which has been transfected with the recombinant vector according to claim 5, and a recombinant vector containing a polynucleotide encoding Cdc42.

8. A protein shown by the amino acid sequence set forth in SEQ ID NO: 2, in the sequence listing.

9. A protein shown by the amino acid sequence set forth in SEQ ID NO: 4 or SEQ ID NO: 6, in the sequence listing.

10. A protein encoded by the polynucleotide according to claim 3 or claim 4.

11. A method of producing the protein according to any one of claims 8 to 10, comprising culturing the transformant according to claim 6 or claim 7.

12. An antibody that recognizes the protein according to any one of claims 8 to 10.

13. A method of identifying a compound that inhibits the function of the proteins according to any one of claims 8 to 10, and/or the expression of the polynucleotides according to any one of claims 1 to 4, comprising detecting the presence, absence or change in the function and/or the expression under conditions where the interaction of a compound with the protein and/or the polynucleotide are allowed, and determining whether the compound inhibits the function of the protein and/or the expression of the polynucleotide.

14. The method according to claim 13, wherein the function of the protein is a guanine nucleotide exchange factor (GEF) activity for Cdc42.

15. A method of determining whether a tissue specimen derived from a human esophageal tissue is a tissue derived from a human esophageal cancer or not, comprising measuring an amount of expression of the polynucleotide according to any one of claims 1 to 4 in the tissue specimen.

16. The method according to claim 15, wherein the method determines that the tissue specimen is a tissue derived from a human esophageal cancer in the case when the amount of expression of the polynucleotide according to any one of claims 1 to 4 in the tissue specimen is 2.5 times higher than that in a control tissue derived from normal human esophagus.

17. An agent for preventing and/or treating an esophageal cancer, comprising a compound that inhibits the function of the protein according to any one of claims 8 to 10 and/or a compound that inhibits the expression of the polynucleotide according to any one of claims 1 to 4, as an active ingredient.

18. A method of preventing and/or treating an esophageal cancer, comprising using a compound that inhibits the function of the protein according to any one of claims 8 to 10 and/or a compound that inhibits the expression of the polynucleotide according to any one of claims 1 to 4.

19. A reagent kit containing at least one selected from the protein according to any one of claims 8 to 10, the polynucleotide according to any one of claims 1 to 4, the recombinant vector according to claim 5, the transformant according to claim 6 or claim 7, and the antibody according to claim 12.
